# EUROPEAN PATENT APPLICATION

(11) **EP 4 431 057 A1**
(43) Date of publication of application: **18.09.2024**
(21) Application number: 23211025.4
(22) Date of filing: 20.11.2023
(51) Int. Cl.: A61F 2/00, A61F 2/04, A61M 27/00

(54) **KIT FOR ENDOURETHRAL PROSTHESIS WITH VALVE**

(30) Priority: 17.03.2023 IT 202300005136
(71) Applicant: RELIEF S.R.L., 56025 Pontedera (PI) (IT)
(72) Inventor: Ricotti, Leonardo, 56037 PECCIOLI (IT); Mazzocchi, Tommaso, 51016 Montecatini Terme (IT); Marziale, Leonardo, 56123 Pisa (IT); Lucarini, Gioia, 55045 Marina di Pietrasanta (IT)
(74) Representative: ABM Agenzia Brevetti & Marchi

(57) **Abstract**

A kit for an endo-urethral prosthesis includes a proximal part (2) and a distal part (4), comprising respective proximal and distal tubular portions (24,46) configured to be arranged within a patient's urethra (7), and respective proximal and distal stents (21,49) configured to anchor internally to a urethra-vesical lumen and urethra, respectively, the proximal and distal tubular portions (24,46) having predetermined radial stiffness, and having, on opposite sides to the proximal and distal stents (21,49), proximal and distal connection ends (29,31) configured to fluid-tightly connect to each other forming a longitudinal duct (5) arranged to convey urine. The distal or proximal tubular portion (46,24) is divided into a first distal or proximal tubular element (35,25) and a second distal or proximal tubular element (45,34) comprising the distal or proximal connection end (31,29), wherein the first distal or proximal tubular element (35,25) and the second distal (45) or proximal tubular element (45,34) have respective mutual fluid-tight connection ends (37,44), the second distal or proximal tubular element (45,25) forming an intermediate part (3), preferably axially flexible, of the prosthesis. In this way, prostheses suitable for the many anatomical variations can be obtained by choosing the length of a single, preferably intermediate, part. In advantageous embodiments, an endo-urethral prosthesis comprises within the proximal and/or distal tubular portion (24,46), at least one valve element (50,150,250) configured to elastically deform, when an opening differential pressure (P*) is exceeded, from a rest closed configuration to a forced open configuration, so that the valve element (50,150,250) can open with an increase in abdominal pressure. In particular, a stopper element (156,256) of an improved valve element (150,250) includes resilient walls (155,255) separated by through slits (157,257) converging from a peripheral portion of the stopper element to a same convergence point (158,258) shifted from the central point of the valve element (150,250) by a predetermined distance (δ) of preferably at least 0,02 mm.

## Description

### Scope of the invention

The present invention relates to an endo-urethral device for treating stenosis of the male urethral duct due to prostatic hypertrophy and/or urinary incontinence conditions in male and female subjects.

### Brief outline of the related art - technical problems

As known, in some cases, prostatic hypertrophy causes stenosis of a longer or shorter portion of the urethra close to the bladder, making it difficult to empty the bladder. It is also known that urination involves a first sphincter known as the internal urethral sphincter, placed immediately outside the bladder outlet, and a second sphincter known as the external urethral sphincter, placed further downstream, with the prostate placed between the two sphincters in the case of male subjects. The external urethral sphincter is made up of striated muscle and its control is voluntary, so as to prevent/allow urination. In general, subjects suffering from prostatic hypertrophy retain the function of the external sphincter and do not suffer from unitary incontinence as a result.

Urinary incontinence conditions are also known to occur in both male and female subjects who are unable to control the external urethral sphincter adequately or at all. Various artificial sphincters exist to treat these conditions, used when remedies such as outpatient therapy, drug therapy and pelvic re-education are not effective. Some artificial sphincters are enclosed by a tubular casing of assigned length for insertion into the urethra, inside of which one or more valve assemblies of various types are arranged, and are described, for instance, in WO2013144770.

Artificial sphincters of the known type, however, have certain drawbacks. In particular, they are not easily adaptable to the anatomical variations of individual patients, which are mentioned below. In other words, the tubular casing of such devices does not always fit the anatomical features associated with such conditions as urinary incontinence and/or prostatic hypertrophy.

A first case concerns incontinent female patients, in whom the urethral duct is short, and who, therefore, require a minimal-length device to limit invasiveness.

A second case concerns male patients who, in addition to suffering from urinary incontinence, also have prostatic hypertrophy problems with stenosis of a longer or shorter portion of the urethra, depending on the size of the prostate in individual patients.

A third important case concerns male patients suffering from urinary incontinence as a result of a post-prostatectomy, an operation that involves a reduction in the length of the urethra. In this case, adapting the device to the new length of the urethra is important to avoid possible pain or other discomfort that the patient may feel during daily life. For instance, a device that is too long might be perceived when the patient sits down. A device that is too short, on the other hand, could lead to lateral leakage of urine and/or migration of the device itself.

Endo-urethral devices are known in which valve assemblies have been used, including resilient walls arranged radially and separated from each other by through slits converging to the centre of the device, configured to switch from a rest closed configuration to a forced open configuration when the urine pressure acting thereon exceeds a predetermined opening pressure, and to resiliently move back to the rest closed configuration once the urine pressure has been released.

Such valve assemblies are described, for example, in the cited patent application WO2013144770, and are particularly cheap and easy to manufacture. However, imperfect opening and closure (the latter causing non-optimal tightness) can sometimes arise with such assemblies. More in detail, an imperfect opening of the valve due to the intravesical pressure can lead to the formation of an open lumen that is too small, which makes it difficult to quickly evacuate the urine. Moreover, in some instances, the resilient walls get stuck together at the moment of the reclosure, which results in a subsequent unvoluntary loss of urine through a residual lumen at the tip of the resilient walls.

In order to mitigate the problem, devices have been proposed in which two of such valve assemblies are serially arranged, or a safety slidable plunger to be magnetically actuated from outside the patient's body has been introduced to maintain the resilient walls in the closed configuration, see WO2013144770. However, such anti-leakage measures complicate the construction and the operation of the device, besides increasing the cost of the same.

The problem of an endo-urethral device, whose length can be adapted by a surgeon according to the real need of the operation, is disclosed in WO2020/089623A2. In particular, a tubular portion is provided at a maximum length, so that an exceeding tubular portion can be cut by the surgeon to adapt to the actual length of the urethra of the patient.

WO2022/180620A1 discloses a short endo-urethral device that is adapted only for male patients. Its length cannot be modified by the surgeon. A proximal tubular end portion of the endo-urethral device can be connected to a tubular extension ending with a stent configured to be anchored in the bladder. Mutual connection means are disclosed of snap-fit type or of positive engagement type between the endo-urethral device and the tubular extension.

### Summary of the invention

It is therefore an object of the present invention to provide an endo-urethral device to counteract stenosis of the urethra in male patients with prostatic hypertrophy, which allows the extent of the stenosis to be taken into account and preserves the functionality of the external urethral sphincter.

It is also an object of the invention to provide an endo-urethral device with an artificial sphincter function that allows for anatomical variations in patients with urinary incontinence.

It is a particular object of the invention to provide an endo-urethral device with the function of an artificial sphincter that can be used in both male and female patients, ensuring minimal invasiveness in the latter case, taking into account the short length of the urethral duct.

It is another particular object of the invention to provide an endo-urethral device with an artificial sphincter function that can be used in male patients, taking into account possible anatomical variations, in particular, in patients with post-prostatectomy urinary incontinence, to avoid the above-mentioned discomforts.

It is also the object of the invention to provide an endo-urethral prosthesis that is capable of curving along with the urethra, so as to minimise the stresses exerted by the prosthesis on the urethra and, with it, pain and possible adverse tissue reactions due to implantation.

It is a further object of the present invention to provide such an endo-urethral device with valve assemblies of the type described In the introduction, which are free from the imperfect opening and closure issues mentioned above.

According to one aspect of the invention, these and other objects are attained by a kit for an endo-urethral prosthesis, comprising a proximal part and a distal part for said endo-urethral prosthesis as defined in claim 1. Advantageous embodiments of the kit are defined in the dependent claims.

The proximal part of the kit comprises:
- a proximal tubular portion configured to be arranged within a patient's urethra, and
- a proximal stent configured to be reversibly fixed to an inner wall of the urethra-vesical lumen;
and the distal part of the kit comprises:
- a distal tubular portion configured to be arranged within the urethra, and
- a distal stent configured to be reversibly fixed to the inner wall of the urethra,

wherein the proximal tubular portion and the distal tubular portion have a predetermined radial stiffness,
wherein the proximal tubular portion and the distal tubular portion have, on sides opposite to the proximal and distal stents, respectively, a proximal connection end and a distal connection end configured to be fluid-tightly connected to each other and so to connect the proximal part and the distal part, thereby defining a single longitudinal duct arranged to convey urine, formed by the proximal tubular portion and the distal tubular portion,
and wherein either the proximal part or the distal part is a single part of an assigned length, and the other part, either distal or proximal, is provided as a range of two or more parts of different lengths, configured to be assembled selectively with the proximal or distal part provided as a single specimen of an assigned length, respectively, so as to make the endo-urethral prosthesis of two or more overall lengths, in order to select one therefrom most appropriate to a patient's urethra.

This way, it is possible to make the endo-urethral prosthesis in a plurality of lengths, so that it can be adapted to the patients' highly variable anatomical features, by making only one part of it in several lengths, in particular the distal part, while the other part is provided in one length, in particular the proximal part, which is more complicate and expensive to manufacture.

In one form of use, the system is an assembly kit delivered as such to the surgeon, comprising one part, either proximal or distal, in a single specimen of an assigned length, and the other part, either distal or proximal, in a plurality of specimens of different lengths, which the surgeon will assemble by taking the components from the kit at the time of implantation to make the endo-urethral prosthesis of a length appropriate to the patient's anatomical features.

As an alternative, the prosthesis of the appropriate length for the individual patient can be ordered and delivered already assembled by the manufacturer or by a seller, once the surgeon has evaluated the length of the patient's urethra and other anatomical parameters.

The execution of only one part of the endo-urethral prosthesis in several lengths makes it possible to obtain an endo-urethral prosthesis of modular length at a reduced cost, and also makes it possible to simplify stockpiling, since it will require the purchase of identical systems.

In particular, according to the invention, assembly kits can be made that can be used for both female patients, who require a minimum length of the endo-urethral prosthesis, and male patients, who may require prostheses of different lengths depending on their anatomical features. This is the case, for instance, of an endo-urethral prosthesis used to resolve a stenosis of the urethra due to compression by a hypertrophic prostate, in which the length of the compressed portion of the urethra may be different from patient to patient, according to the size of the prostate.

With respect to WO2020/089623A2, where an exceeding portion of a tubular catheter or external retention mechanism can be cut, it is avoided that cut portions that may contain small particles can enter the body of the patient. Moreover, contrarily to WO2020/089623A2, where after cutting a portion the proximal or distal stents cannot be easily provided, in the invention the terminal stents can be maintained as provided by the manufacturer without any alterations.

Preferably, the proximal tubular portion and/or the distal tubular portion has a diameter set between 3 and 10 mm, and a wall thickness set between 0,2 and 0,8 mm.

As an alternative, the distal part is provided as a range of two or more parts of different lengths and the distal tubular portion is split into a first distal tubular element of an assigned length, and a second distal tubular element provided as a range of two or more elements of different lengths, the second distal tubular element comprising the distal connection end, wherein the first distal tubular element and the second distal tubular element have respective mutual fluid-tight connection ends, the second distal tubular element forming an intermediate part of the endo-urethral prosthesis. Similarly, as an alternative, the proximal part is provided as a range of two or more parts of different lengths and the proximal tubular portion is split into a first proximal tubular element of an assigned length and a second proximal tubular element provided as a range of two or more elements of different lengths, the second proximal tubular element comprising the proximal connection end, wherein the first proximal tubular element and the second proximal tubular element have respective mutual fluid-tight connection ends, the second proximal tubular element forming an intermediate part of the endo-urethral prosthesis.

In other words, the prosthesis is made in three parts coupled to each other, whereby only the intermediate part can advantageously be made in several pieces of different lengths in order to obtain the different lengths of the prosthesis. This makes even more straightforward and cheaper to cope with the anatomical differences from patient to patient by means of endo-urethral prostheses whose length can be defined at the time of implantation, thanks to the simple construction of the intermediate part, which can be a simple small tube with two connection end portions.

In one embodiment, a valve element is fluid-tightly arranged within at least one portion selected between the proximal tubular portion and the distal tubular portion, the valve element including a stopper portion, wherein the stopper portion comprises resilient walls configured to be deformed:
- from a rest closed configuration of the valve element, in which the urine cannot flow through the longitudinal duct,
- to a forced open configuration of the valve element, in which the urine can flow through the longitudinal duct,
when a differential pressure acting on the resilient walls exceeds a predetermined opening pressure of the valve element. In this way, the patient can change the configuration of the valve element from the closed configuration to the open configuration by causing his/her abdominal pressure to increase, i.e., through a contraction of the abdominal muscles.

This way, an endo-urethral prosthesis is obtained to treat urinary incontinence that a conscious patient can control by an abdominal effort strong enough to appropriately modify the intravesical pressure, i.e., to increase the latter above the opening pressure of the valve assembly. The opening pressure of the valve assembly will be set to a value lower than a pressure that could damage the bladder and the organs and ducts connected thereto.

Advantageously, the resilient walls of the stopper portion are separated from each other by through slits, wherein:
- in the closed configuration, the stopper portion has a shape selected between a flat shape and a convex shape with a convexity oriented towards the proximal stent, in such a way that a urine pressure acting on the stopper portion keeps the through slits closed;
- in the open configuration, the stopper portion has a concave shape, in particular opposed to the possible convex shape of the stopper portion, wherein the through slits are deformed and open, and is configured to allow a flow of urine,
wherein, in the closed configuration, the stopper portion is configured to withstand the urine pressure until said urine pressure does not exceed said opening pressure, so that, upon exceeding said opening pressure, the stopper portion collapses into the concave shape.

The remarkable simplicity of manufacture, in particular the ease with which the opening pressure and reclosing pressure of such a valve element can be predetermined, makes its use in the context of the present invention particularly advantageous.

In particular, the stopper portion has a dome shape with a through-slot converging to a central region of the stopper portion.

In a preferred exemplary embodiment of the kit,
the valve element comprises a peripheral cylindric montage portion fluid-tightly mounted to an internal wall of the at least one tubular portion,
the stopper portion of the valve element has a central point which is the centre with respect to the cylindric montage portion,
and the through slits converge to a same convergence point that is shifted from the central point by a predetermined distance of preferably at least 0,02 mm.

In particular,
the stopper portion comprises a peripheral support disc from which the resilient walls centripetally extend towards the central point,
the valve element comprises a connection portion between the peripheral support disc and the montage portion,
and the peripheral support disc is selected between:
   - a peripheral support disc concentric to the peripheral cylindric montage portion, and the stopper portion is manufactured eccentrically to the peripheral support disc;
   - a peripheral support disc eccentric to the peripheral cylindric montage portion, and the stopper portion is manufactured concentrically to the peripheral support disc.

Advantageously, two annular sealing elements protrude from the outer surface of the intermediate part of the endo-urethral prosthesis, preferably arranged at the connection ends of the intermediate part. This improves the tightness of the endo-urethral prosthesis and also improves stabilisation along the urethra.

In an advantageous construction form, the intermediate part of the endo-urethral prosthesis is axially flexible, i.e., it is configured to move:
- from a straight conformation, in which the distal part is aligned with the proximal part, i.e., the distal and proximal parts have substantially coincident longitudinal axes,
- to a flexed configuration, in which the proximal and distal parts are connected together by an elbow portion.

This allows the endo-urethral prosthesis to curve together with the natural urethra, reducing the non-physiological stresses exerted on the latter and, therefore, the pain associated with such stresses and ultimately the possible adverse tissue reactions due to the implantation of the prosthesis.

In particular, the intermediate part is at least partially made of a biocompatible flexible polymeric material, more in particular, it is made of a medical-grade silicone.

In an advantageous embodiment, the intermediate part of the endo-urethral prosthesis comprises:
- two rigid connection end portions for connection to:
   - the proximal part and the first distal tubular element, or
   - the first proximal tubular element and the distal part;
- a central axially flexible tubular portion made of a polymeric material with a predetermined elasticity modulus, selected among the biocompatible flexible materials, in particular, among medical-grade silicones.

In particular, the two rigid connection end portions comprise respective cylindrical extensions moulded within a material of the thickness of the axially flexible central tubular portion.

These exemplary embodiments and modification make it possible to produce an axially flexible endo-urethral prosthesis in a relatively easy way.

In another embodiment, a first valve element or proximal valve element and, distally with respect to it, a second valve element or distal valve element are fluid-tightly arranged within the longitudinal duct. In this way, it is possible to limit the possibility that the endo-urethral prosthesis may open undesirably under certain circumstances, for example, due to heavy coughing or inertial actions that may occur while running up or down a flight of stairs quickly.

In particular, the proximal valve element has a reclosing pressure higher than the opening pressure of the distal valve element. Thus, the section of the longitudinal canal of the endo-urethral prosthesis that lies between the proximal and distal valve elements can be cleared of urine at the end of urination.

According to another aspect of the invention, the above-mentioned objects are reached by an endo-urethral prosthesis comprising a proximal part and a distal part,
wherein the proximal part comprises:
   - a proximal tubular portion configured to be arranged within a patient's urethra, and
   - a proximal stent configured to be reversibly fixed to an inner wall of a urethra-vesical lumen;
wherein the distal part comprises:
   - a distal tubular portion configured to be arranged within the urethra, and
   - a distal stent configured to be reversibly fixed to the inner wall of the urethra,
the proximal tubular portion and the distal tubular portion having a predetermined radial stiffness and defining a longitudinal duct arranged to convey urine;
wherein a valve element is fluid-tightly arranged within at least one portion selected between the proximal tubular portion and the distal tubular portion, the valve element including a stopper portion, wherein the resilient walls are configured to be deformed:
   - from a rest closed configuration of the valve element, in which the urine cannot flow through the longitudinal duct,
   - to a forced open configuration of the valve element, in which the urine can flow through the longitudinal duct,
when a differential pressure acting on the resilient walls exceeds a predetermined opening pressure of the valve element, so that the patient can change the configuration of the valve element from the closed configuration to the open configuration by causing his/her abdominal pressure to increase; wherein the resilient walls of the stopper portion are separated from each other by through slits, wherein:
   - in the closed configuration, the stopper portion has a shape selected between a flat shape and a convex shape with a convexity oriented towards the proximal stent, in such a way that a urine pressure acting on the stopper portion keeps the through slits closed;
   - in the open configuration, the stopper portion has a concave shape, in particular opposite to the convex shape, wherein the through slits are deformed and open, and is configured to allow a flow of urine,
wherein, in the closed configuration, the stopper portion is configured to withstand the urine pressure until the urine pressure does not exceed the opening pressure, so that, upon exceeding the opening pressure, the stopper portion collapses into the concave shape,
wherein the valve element comprises a peripheral cylindric montage portion fluid-tightly mounted to an internal wall of the at least one tubular portion,
wherein the stopper portion of the valve element has a central point which is the centre with respect to the cylindric montage portion,
and wherein the through slits converge to a same convergence point that is shifted from the central point by a predetermined distance.

This way, the resilient walls have respective areas slightly different from one another, therefore the resilient walls receive respective opening forces different from one another as well. Moreover, since the areas of the resilient walls are different from one another, the resilient walls also have a deformation energy, i.e., the minimum energy they must receive to become deformed with respect to their rest closed configuration shape and, upon returning to their rest closed configuration, they do not get stuck against each other.

In particular,
the stopper portion comprises a peripheral support disc from which the resilient walls centripetally extend towards the central point,
the valve element comprises a connection portion between the peripheral support disc and the montage portion,
and the peripheral support disc is selected between:
   - a peripheral support disc concentric to the peripheral cylindric montage portion, and the stopper portion is manufactured eccentrically to the peripheral support disc;
   - a peripheral support disc eccentric to the peripheral cylindric montage portion, and the stopper portion is manufactured concentrically to the peripheral support disc.

Preferably, the proximal tubular portion and/or the distal tubular portion has a diameter set between 3 and 10 mm, and a wall thickness set between 0,2 and 0,8 mm.

In particular, the convergence point and the central point are at a distance of at least 0,02 mm.

### Brief description of the drawings

The invention is illustrated below by a description of some exemplary embodiments, by way of example and not limitation, with reference to the accompanying drawings, in which
- Figs. 1 and 2 diagrammatically show an endo-urethral prosthesis arranged in the urethra of a female and male patient, respectively, suffering from urinary incontinence;
- Fig. 3 diagrammatically shows a condition of urethral stenosis in a male patient suffering from prostatic hypertrophy;
- Fig. 4 diagrammatically shows an endo-urethral prosthesis placed in the urethra of the patient in Fig. 3, in order to resolve the urethral stenosis;
- Fig. 5 is a diagrammatic axonometric view of a kit including one specimen of a proximal portion of the endo-urethral prosthesis and a plurality of specimens of different lengths of a distal portion of the endo-urethral prosthesis, according to an embodiment of the invention;
- Figs. 6 and 7 are diagrammatic longitudinal exploded cross-sectional views of composite kits such as the kit in Fig. 5 and comprising one and two valve elements, respectively, arranged within the longitudinal channel;
- Figs. 8-10 are diagrammatic axonometric, top and longitudinal cross-sectional views, respectively, of a valve element of the kit according to an exemplary embodiment of the invention, in which, in the closed configuration, the stopper portion has a flat shape;
- Figs. 11-13 are diagrammatic axonometric, top and longitudinal cross-sectional views of a valve element of another exemplary embodiment of the invention, in which, in the closed configuration, the stopper portion has a convex shape, in particular, a dome shape, with the convexity oriented towards the proximal end;
- Figs. 14 and 15 are diagrammatic longitudinal exploded cross-sectional views of a kit in which the distal part and the proximal part of the endo-urethral prosthesis, respectively, are made in two pieces, and comprise an intermediate part of the endo-urethral prosthesis that is provided in a plurality of specimens of different lengths, according to respective exemplary embodiments of the invention;
- Fig. 16 is a diagrammatic longitudinal exploded cross-sectional view of a kit composed like the kit of Fig. 14 or the kit of Fig. 15, and including a valve element within the longitudinal channel;
- Fig. 17 is a diagrammatic longitudinal exploded cross-sectional view of a kit composed like the kit of Fig. 14 or the kit of Fig. 15, and including two valve elements within the longitudinal channel;
- Fig. 18A is a diagrammatic exploded axonometry view of composite kits as in Figs. 14-17;
- Figs. 18B and 18C are diagrammatic perspective and lateral views, respectively, of a connecting element to connect the proximal or distal stent, on the one hand, and the prosthesis body, on the other hand.
- Fig. 19 is a diagrammatic side view of a mounted endo-urethral prosthesis that can be obtained from the three-piece kits of Figs. 14 to 17 according to a modification of a respective exemplary embodiment in which the intermediate part also includes radial sealing elements;
- Fig. 20 is a diagrammatic longitudinal sectional view of the intermediate part of the kit of Fig. 19;
- Fig. 21 is a diagrammatic longitudinal cross-sectional view of an intermediate part of a kit as in Figs. 14 to 17, according to an exemplary embodiment;
- Figs. 22 and 23 are diagrammatic side and axonometric views, respectively, of the rigid connection end portions of the intermediate part of Fig. 21;
- Fig. 24 is a diagrammatic side view of a mounted endo-urethral prosthesis that can be obtained from one of the three-piece kits of Figs. 14 to 17, according to an exemplary embodiment in which the intermediate part is axially flexible;
- Fig. 25 is a diagrammatic exploded axonometric view of kits such as the kits of Figs. 14 to 17, according to modifications of the respective exemplary embodiments, in which the proximal, intermediate and distal parts have snap-on connection ends instead of threaded connection ends;
- Fig. 26 is a diagrammatic longitudinal sectional view of a proximal part of a device according to a modification of the exemplary embodiment shown in Fig. 15, in which the proximal part and the intermediate part have snap-on connection ends instead of threaded connection ends;
- Fig. 27 is a diagrammatic longitudinal cross-sectional view of the proximal tubular portion of the endo-urethral prosthesis of Fig. 26;
- Fig. 28 diagrammatically shows an endo-urethral prosthesis arranged in the urethra of a female animal, typically a dog, suffering from urinary incontinence;
- Figs. 29-31 are perspective, top and cross-sectional views of a draft to make a valve element in which a stopper portion includes resilient walls separated from each other by through slits, before the slits are punched, according to the prior art;
- Figs. 32-34 are perspective, top and cross-sectional views of a draft to make a valve element in which a stopper portion includes resilient walls separated from each other by through slits, before the slits are punched, in one exemplary embodiment of the present invention;
- Figs. 35-37 are perspective, top and cross-sectional views, respectively, of a valve element in which a stopper portion includes resilient walls separated from each other by through slits, according to the same prior art of Figs. 29-31;
- Figs. 38-40 are perspective, top and cross-sectional views, respectively, of a valve element in which a stopper portion includes resilient walls separated from each other by through slits, in the same embodiment as in Figs. 32-34;
- Figs. 41 and 42 are perspective views of the prior art valve elements of Figs. 35-37 and of the inventive valve elements of Figs. 38-40, respectively, in which the surfaces are highlighted upon which the urine pressure is exerted;
- Fig. 43 is a diagram showing how the urine pressure acting on the resilient walls of such valve elements as in Figs. 35-40 changes with time before, during and after the physiological opening of the valve itself;
- Figs. 44a-44e are diagrammatical perspective views of such a prior art valve as in Figs. 35-37, showing how the shape of the stopper portion changes with time during the opening event referred to in Fig. 43;
- Figs. 45a-45e are diagrammatical perspective views of such an inventive valve as in Figs. 38-40, showing how the shape of the resilient walls changes with time during the opening event referred to in Fig. 43;
- Fig. 46 is a perspective view of a prior art valve element as in Figs. 35-37;
- Fig. 47 is a cross-sectional view of a valve element according to a further exemplary embodiment of the present invention, in which a stopper portion includes resilient walls separated from each other by through slits;
- Figs. 48 and 49 are lateral views of one-piece endo-urethral devices including valve elements as in Figs. 38-40.

### Description of preferred embodiments

The following is a description of some exemplary embodiments, and modifications thereof, of a kit, i.e. a system, for an endo-urethral prosthesis 1 according to an aspect of the invention, suitable for treating male and female patients with urinary incontinence, and male patients with prostatic hypertrophy resulting in stenosis of the urethra 7 at the prostate 9, and of an endo-urethral prosthesis 1'.

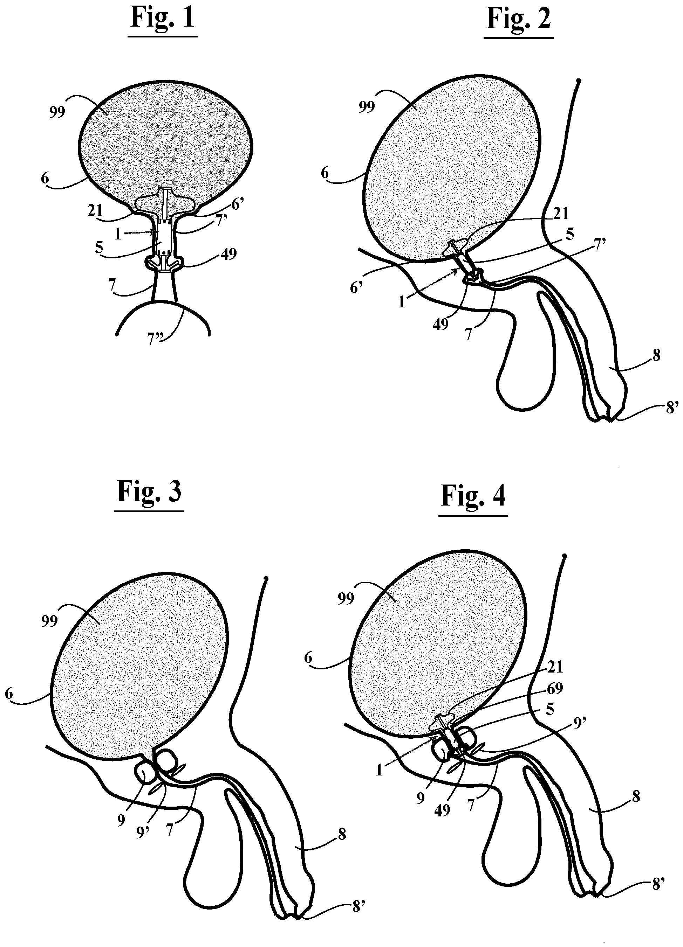

As shown in Figs. 1, 2 and 4, endo-urethral prosthesis 1 is configured to be arranged within urethra 7 of a female patient (Fig. 1) or a male patient (Figs. 2 and 4), preferably at the connection between bladder 6 and urethra 7. Endo-urethral prosthesis 1 comprises a longitudinal endo-urethral duct 5 suitable for conveying urine 99 contained in bladder 6, as well as fixation means 21,49 for stably but reversibly arrange endo-urethral prosthesis 1 in a predetermined position within urethra 7.

The fixation means to urethra 7 preferably comprise a proximal stent 21 and a distal stent 49. In this description, the adjectives "proximal" and "distal" refer to the patient wearing endo-urethral prosthesis 1.

Proximal stent 21 and distal stent 49 are configured to elastically deform from an expanded rest configuration, shown in the figures, in which they have respective radial dimensions larger than the natural radial dimension of urethra 7, to a contracted forced configuration, in which the radial dimensions of proximal stent 21 and distal stent 49 can be elastically constricted in such a way that endo-urethral prosthesis 1 can be inserted into and slid within the lumen of urethra 7, until a predetermined implantation position of endo-urethral prosthesis 1 within urethra 7 is reached.

In the non-limiting embodiment shown in the figures, proximal stent 21 is configured to elastically anchor itself to inner wall 6' of bladder 6, at the mouth of urethra 7, so as to prevent endo-urethral prosthesis 1, after implantation, from moving in a distal direction and ultimately being ejected from urethra 7.

More in particular, in the embodiment shown in the figures, proximal stent 21 may comprise a plurality of elongated anchor elements 22, each having a first end attached to a circumferential position of a continuous proximal connecting ring 27a and a second end attached to a respective ring segment, the flexible ring segments forming a connecting ring 27b for connection to the body of endo-urethral prosthesis 1, for example in the manner further described below. Elongated anchor elements 22 are elastically flexible between a flexed rest conformation, shown in the figures, corresponding to the expanded rest configuration of proximal stent 21, and an extended forced conformation, not shown, corresponding to the contracted forced configuration of proximal stent 21.

In this embodiment, distal stent 49 is configured to elastically anchor to the inner wall 7' of urethra 7, so as to prevent endo-urethral prosthesis 1, after implantation, from moving in a proximal direction and ultimately entering bladder 6.

More in particular, in the embodiment shown in the figures, distal stent 49 can comprise a plurality of radial anchoring elements 48, each radially protruding from a central element and elastically movable from an expanded rest position, shown in the figures, corresponding to the expanded rest configuration of distal stent 49, and a contracted forced position, not shown, corresponding to the contracted forced configuration of distal stent 49. The central element of distal stent 49 comprises a plurality of elongated flexible portions, each having one end attached to a respective ring segment, the flexible ring segments forming a connecting ring 48a for connection to the body of endo-urethral prosthesis 1, for example, in the manner further described below.

More in particular, Fig. 1 refers to an implantation of endo-urethral prosthesis 1 in the urethra of a female patient, in which urethra 7 extends between patient's bladder 6 and patient's vulvar vestibule 7', while Fig. 2 refers to an implantation of endo-urethral prosthesis 1 in urethra 7 of a male patient, in which urethra 7 extends between patient's bladder 6 and patient's glans 8' through patient's penis 8.

Fig. 3 diagrammatically shows a condition of stenosis of urethra 7 in a male patient due to hypertrophy of prostate 9, and also shows, downstream of the region of urethra 7 affected by the stenosis, the striated muscle that presides over the control of a natural external urethral sphincter 9'. Fig. 4, instead, diagrammatically shows endo-urethral prosthesis 1 used to resolve the stenosis. In this case, endo-urethral prosthesis 1 has such a length that it does not interfere with natural external urethral sphincter 9, whose functionality may need to be preserved in patients who do not suffer from urinary incontinence. For this purpose, endo-urethral prosthesis 1 has a length that can be modulated, which is made possible by the kit according to the invention, in the manner described below.

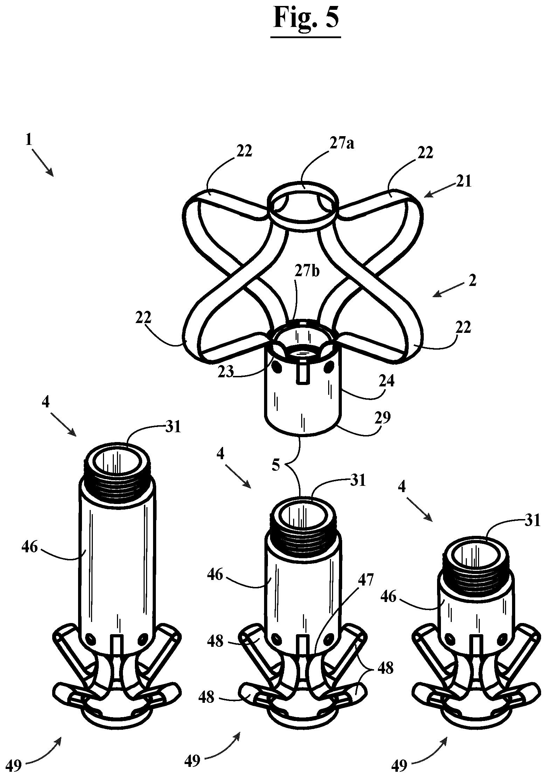

With reference to Figs. 5-7, a kit including a proximal part 2 and a distal part 4 of endo-urethral prosthesis 1 is described in three different exemplary embodiments.

In the kit of Figs. 5-7, proximal part 2 is provided in a single piece of length L₂, while distal part 4 is provided in a plurality of pieces of different lengths L₄. In the figures, three pieces 4 are shown in a non-limiting manner, in other words, 2, 3, 4... n pieces 4 of different lengths L₄ can be provided in the kit. In this way, it is possible to assemble an endo-urethral prosthesis 1 that can have different lengths L₁ corresponding to the combination of lengths L₂ and L₄, respectively, of the one proximal part 2 and the one proximal part 4, selected according to the requirements of the implant to be performed, mainly depending on the patient's anatomical specific features.

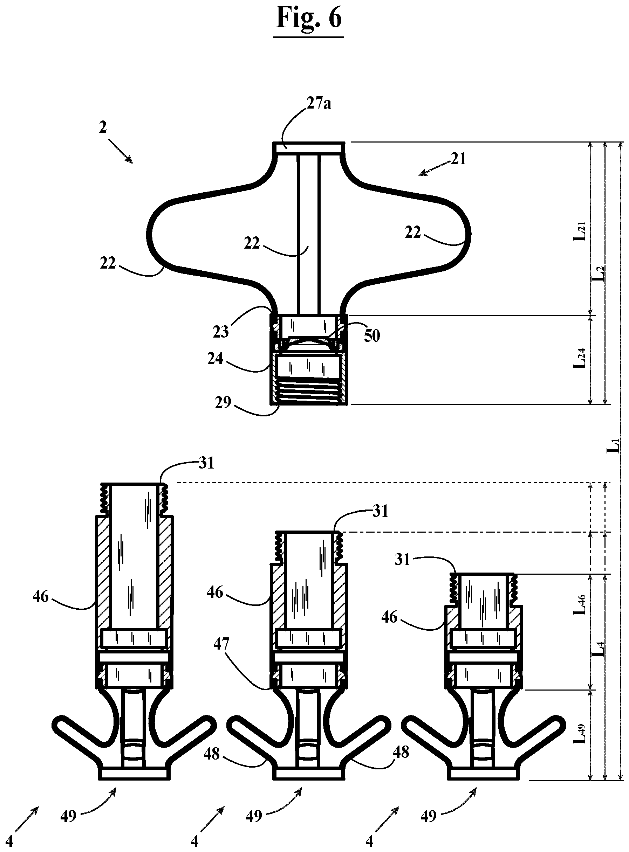

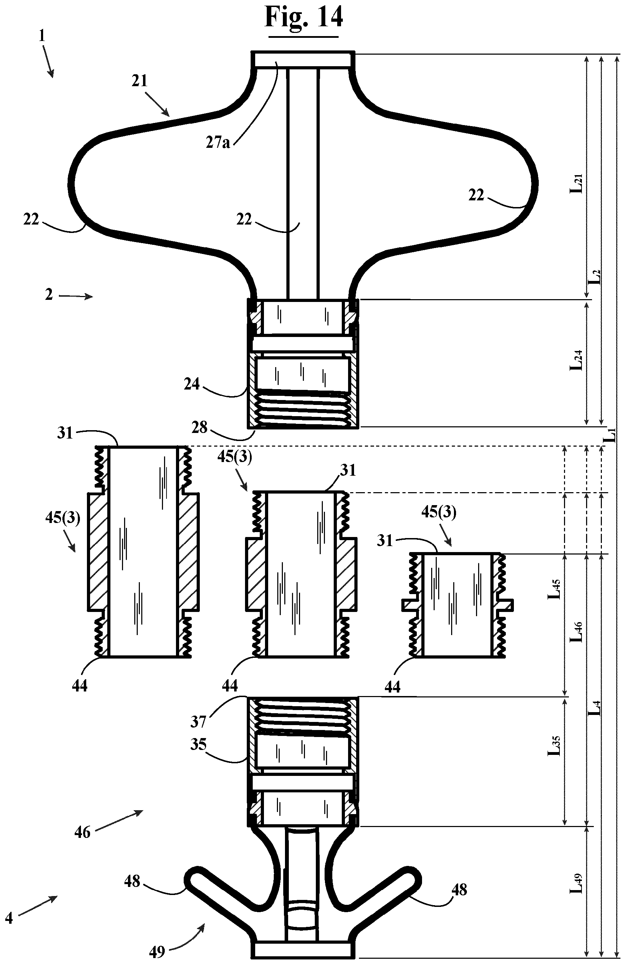

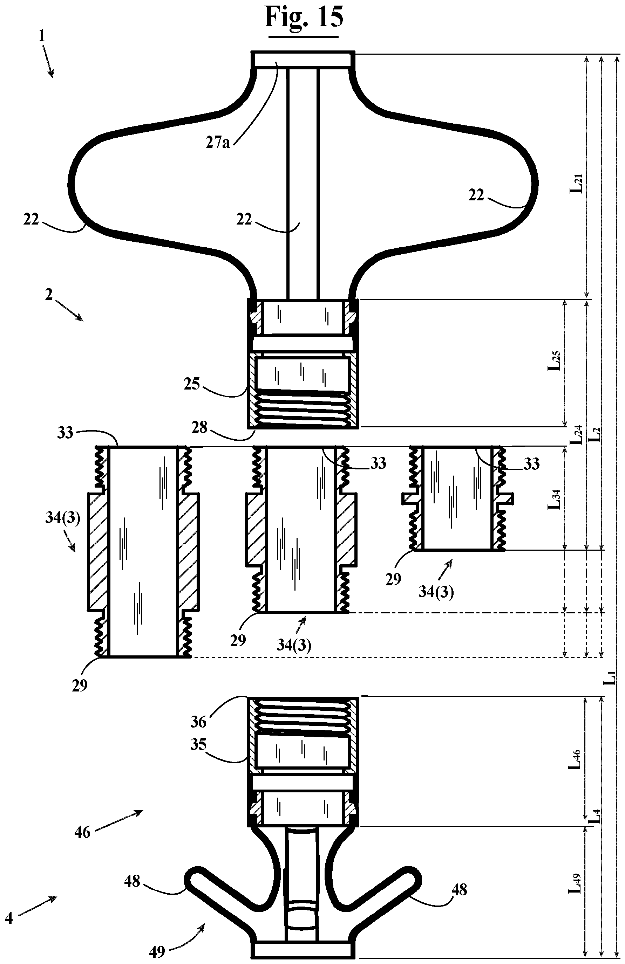

Figs. 6,7,14,15 are longitudinal exploded cross-sectional views of composite kits. The length of endo-urethral prosthesis 1 in all these pictures, of distal part 4 in Fig 14 and of proximal part 2 in Fig. 15, are represented conventionally for the sake of clarity, including also the "empty" spaces due to the exploded representation of the cited parts.

A first case of anatomical variant can be deduced from Figs. 3 and 4, in which the stenosis of urethra 7 due to the compression by hypertrophic prostate 9 can have various lengths depending on the size of patient's prostate 9, just as the distance of external urethral sphincter 9 from the opening of urethra 7 can have different values. A second, more general case of anatomical variant concerns female patients, in whom the urethra is shorter, and it is convenient that endo-urethral prosthesis 1 has a minimum length, so that the implantation is minimally invasive.

Obviously, according to an embodiment, not shown, assembly kits are encompassed in which distal part 4 is provided as a single piece of length L₄, while proximal part 2 is provided as a plurality of pieces of different lengths L₂, or even in which both proximal part 2 and distal part 4 are both provided in a plurality of pieces of different lengths L₂ and L₄.

More in detail, proximal part 2 comprises proximal stent 21 of length L₂₁, for example in the form previously described for anchoring to inner walls 6' and 7' of bladder 6 and urethra 7, at the internal urethral sphincter, and a proximal tubular portion 24 of length L₂₄, also suitable for arrangement within urethra 7. Similarly, each of the specimens of distal part 4 comprises previously mentioned distal stent 49 having a length L₄₉, and a distal tubular portion 46 having a length L₄₆, also suitable for arrangement within urethra 7. Proximal tubular portion 24 and distal tubular portion 46 have a predetermined radial stiffness and together form longitudinal endo-urethral duct 5 of endo-urethral prosthesis 1, which is rigid enough to maintain its shape under the implantation conditions, for example under the implantation conditions of Fig. 4, in which hypertrophied prostate 9 exerts constricting forces on the outer walls of urethra 7, but also under other patient's substantially permanent conditions, for example presence of excessive fatty tissue.

Preferably, proximal tubular portion 24 and distal tubular portion 46 and therefore longitudinal duct 5 has a diameter set between 3 and 10 mm, and a wall thickness set between 0,2 and 0,8 mm.

Proximal tubular portion 24 and distal tubular portion 46 have distal and proximal ends 23 and 47 connecting to proximal stent 21 and distal stent 49, respectively. Opposite ends 23 and 47, proximal tubular portion 24 and distal tubular portion 46 have a proximal connection end 29 and a distal connection end 31, respectively, configured to be fluid-tightly connected to each other and connect therefore proximal part 2 and distal part 4, so as to form endo-urethral duct 5.

In the embodiment shown in Fig. 5, endo-urethral duct 5 is arranged to always allow a flow of urine 99 therethrough. In other words, endo-urethral prosthesis 1, in this case, does not include any flow-stop means, and is suitable for applications of the type shown in Fig. 4, to treat stenosis of urethra 7 due to hypertrophic prostate 9. Length L₁ is short enough to prevent external urethral sphincter 9 from reaching natural external urethral sphincter 9, and is obtained by appropriately selecting the piece of suitable length L₄ as the distal part of endo-urethral prosthesis 1 itself, when assembling the latter.

On the other hand, endo-urethral duct 5 of endo-urethral prosthesis 1 according to the embodiment shown in Fig. 6, encloses a valve element 50 that is arranged to stop or allow the flow of urine 99 through endo-urethral duct 5 in a rest closed configuration and in a forced open configuration, respectively.

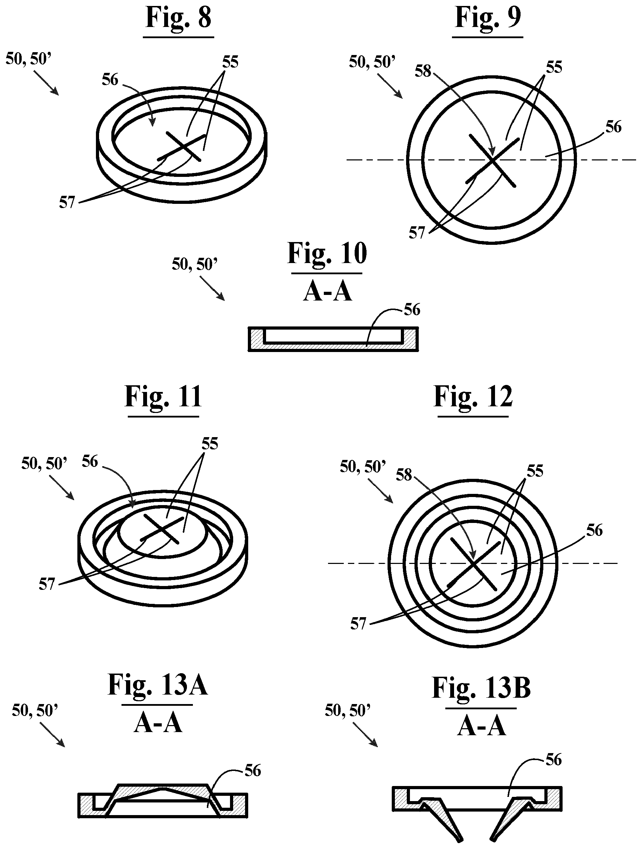

Figs. 8-10 and 9-11 show two exemplary valve elements 50, in which a stopper portion 56 comprises resilient walls 55 configured to deform from the rest closed configuration, shown in the figures, to the forced open configuration, when a differential pressure ΔP acting on resilient walls 55 exceeds a predetermined characteristic opening pressure P* of valve element 50.

Endourethral prosthesis 1 according to the exemplary embodiment shown in Fig. 6, and other embodiments described below, is suitable for treating both conscious and unconscious male or female patients suffering from urinary incontinence.

In particular, a conscious patient wearing endo-urethral prosthesis 1 is able to bring the configuration of valve element 50 from the closed configuration to the open configuration by a voluntary abdominal effort in which the abdominal pressure is increased. Once this effort is released, resilient walls 55 resiliently move back to the rest closed configuration of valve element 50, thus allowing urine 99 to physiologically accumulate within bladder 6 again for a new cycle, without any leakage.

On the other hand, in an unconscious patient, such a valve element 50 allows physiological emptying of bladder 6 when the intravesical pressure exceeds a critical value substantially equal to opening pressure P*, thus preventing the bladder and the organs and ducts hydraulically connected thereto from being damaged by excessive internal overpressure. Obviously, this is possible provided that opening pressure P* has been appropriately predetermined, through a suitable choice of the physical and geometric characteristics of valve element 50, in particular of resilient walls 55.

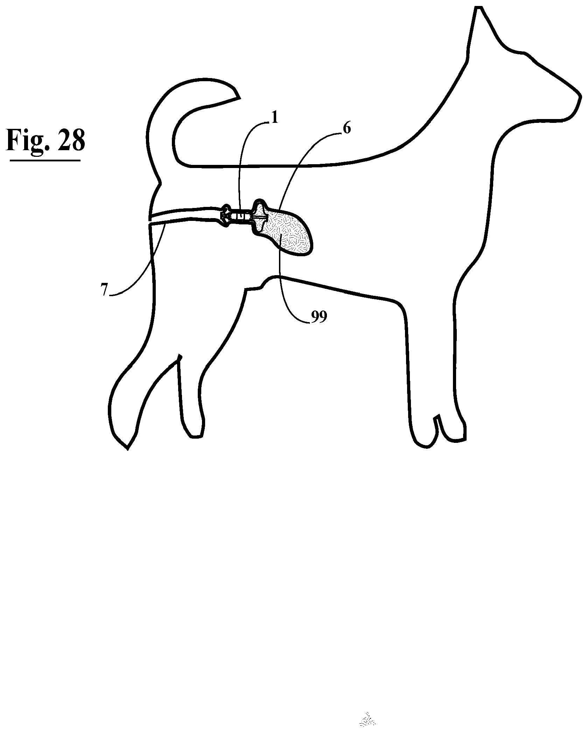

Since endourethral prosthesis 1 in Fig. 6 is controlled by a physiological increase of the intravesical pressure, such a device is suitable for treating animals with urinary incontinence, typically female animals, as diagrammatically shown in Fig. 28.

Valve element 50 of the kit of Fig. 6 is arranged within proximal part 2 of endo-urethral prosthesis 1, in particular it is arranged in proximal tubular portion 24. However, in an embodiment modification not shown, it may be arranged within distal part 4, in particular within distal tubular portion 46.

In valve elements 50 of the embodiments shown in Figs. 8 to 13B, resilient walls 55 are arranged radially and are separated from each other by through slits 57, in this case converging to a preferably central region 58 of stopper portion 56 of valve element 50.

In particular, in the closed configuration, stopper portion 56 of valve element 50 of Figs. 8-10 has a flat shape, whereas stopper portion 56 of valve element 50 of Figs. 9-13B has a convex shell shape, for example a dome shape, not shown, with the convexity pointing to the bladder side, i.e. oriented towards proximal stent 21, and forms a diaphragm such that urine pressure 99 acting over convex shell shape 56 keeps through slits 57 closed, provided that the urine pressure does not exceed opening pressure P*. In other words, stopper portion 56 is configured to withstand such urine pressure until the urine pressure reaches opening pressure P*. Upon reaching opening pressure P*, stopper portion 56 collapses into a concave shape, opposite to the convex shape, in which through slits 57 are deformed and stopper portion 56 is open as shown in Fig. 13B, so as to allow the flow of urine 99 through longitudinal duct 5 and therefore through urethra 7.

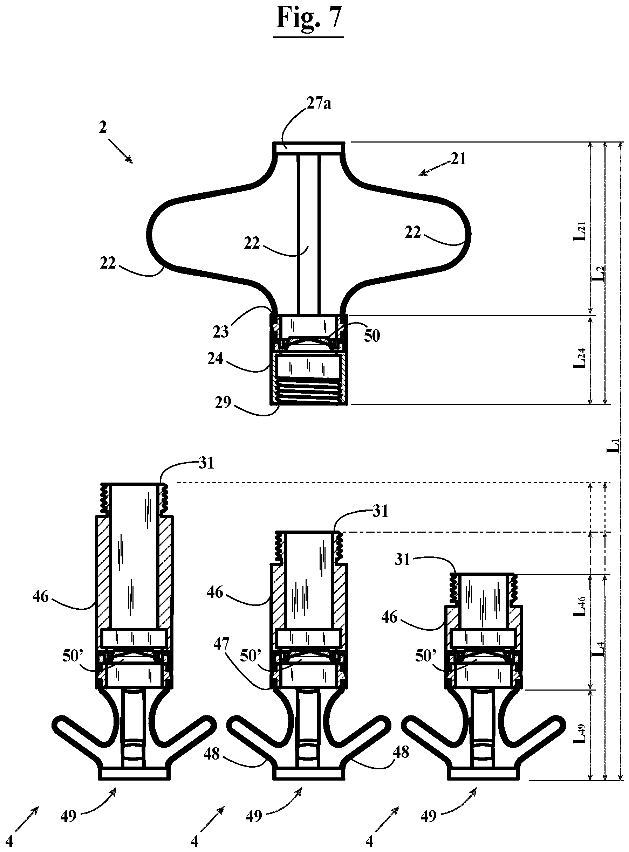

Fig. 7 shows a kit according to another embodiment, which differs from the kit of Fig. 6 in that it comprises two valve elements 50,50', instead of one. In the modification shown in the figure, a proximal valve element 50 is arranged within proximal part 2, in particular in proximal tubular portion 24, and a distal valve element 50' is arranged within distal part 4, in particular in distal tubular portion 46. In unshown modifications of such a kit, however, two valve elements 50,50' may both be arranged in proximal part 2 or both in distal part 4. Preferably, in these cases, proximal valve element 50 has a reclose pressure higher than the opening pressure of distal valve element 50', so that a section of longitudinal duct 5 between proximal valve element 50 and distal valve element 50' is cleared of urine 99 after evacuation of the same.

With reference to Fig. 14, a kit including a proximal portion 2 and a distal portion 4 of endo-urethral prosthesis 1 is described, according to an embodiment of the invention, which differs from the kit of Fig. 6 in that distal tubular portion 46 is in turn split into a first distal tubular element 35 and a second distal tubular element 45 comprising a distal end 31 for connection to proximal part 2. First distal tubular element 35 and second distal tubular element 45 have respective liquid-tight mutual connection ends 37,44.

First distal tubular element 35 is provided in a single piece of length L₃₅, while second distal tubular element 45 is provided in a plurality of pieces of different lengths L₄₅. For this reason, distal tubular portion 46 is provided in a same plurality of pieces of different lengths L₄₆ corresponding to the combination of the different lengths L₄₅ of second distal tubular element 45 with length L₃₅ of first distal tubular element 35, and distal part 4 is therefore provided in an equal plurality of pieces of different lengths L₄ corresponding to the combination of the different lengths L₄₆ of distal tubular portion 46 with length L₄₉ of distal stent 49. Three pieces 45 are non-limitatively shown in the figure, in other words 2, 3, 4... n pieces 45 of different lengths L₄₅ may be provided.

With reference to Fig. 15, a kit including a proximal portion 2 and a distal portion 4 of endo-urethral prosthesis 1 is described according to an embodiment of the invention, in which proximal part 2 is provided in a plurality of pieces of different lengths L₂, while distal part 4 is provided in a single piece of length L₄. Proximal tubular portion 24 is in turn split into a first proximal tubular element 25 and a second proximal tubular element 34 comprising proximal connection end 29 for connection to distal part 4. First proximal tubular element 25 and second proximal tubular element 34 have respective liquid-tight mutual connection ends 28,33.

First proximal tubular element 25 is provided in a single piece of length L₂₅, while second proximal tubular element 34 is provided in a plurality of pieces of different lengths L₃₄. For this reason, proximal tubular portion 24 is provided in a same plurality of pieces of different lengths L₂₄ corresponding to the combination of the different lengths L₃₄ of second proximal tubular element 34 with length L₂₅ of first proximal tubular element 25, and proximal part 2 is provided in an equal plurality of pieces of different lengths L₂ corresponding to the combination of the different lengths L₂₄ of proximal tubular portion 24 with length L₂₁ of proximal stent 21. Three pieces 34 are non-limitatively shown in the figure, in other words 2, 3, 4... n pieces 34 of different lengths L₃₄ may be provided.

In the kits of Figs. 14 and 15, second distal tubular element 45 and first distal tubular element 34, in the various lengths, respectively, form an intermediate part 3 of the endo-urethral prostheses 1 that can be obtained from such kits. Thus, from another point of view, Figs. 14 and 15 refer to a kit including a proximal part 2 or 21-24, an intermediate part 3 and a distal part 35-49 or 4 of endo-urethral prosthesis 1, wherein intermediate part 3 is provided in a plurality of pieces of different lengths L₃.

Endo-urethral prostheses 1 that can be obtained from the kits shown in Figs. 14 and 15 has longitudinal duct 5 that does not include any flow-stop means, and therefore is arranged to always allow urine 99 to flow therethrough, so it is suitable for treating stenosis of urethra 7 due to prostatic hypertrophy, similarly to the endo-urethral prosthesis that can be obtained from the kit shown in Fig. 3.

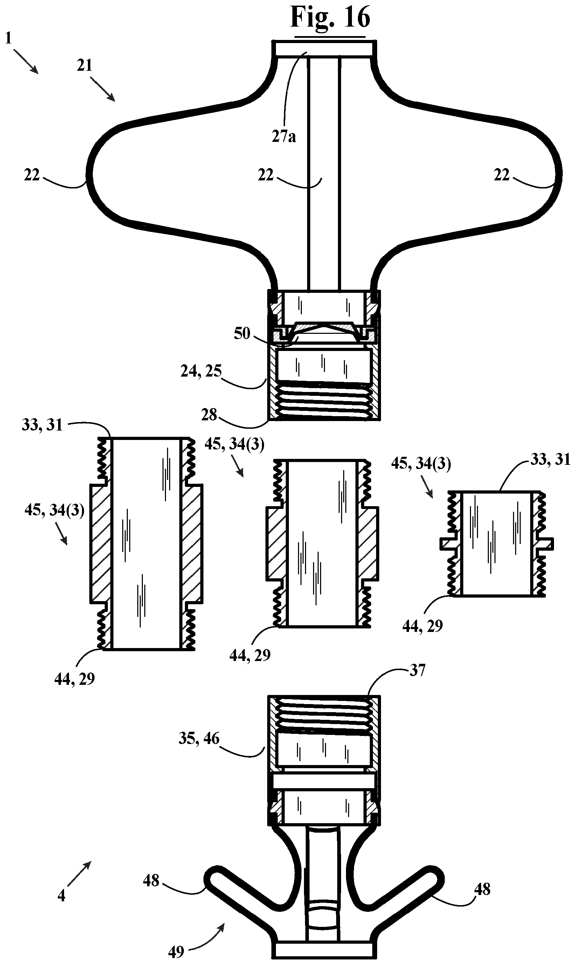

The kit shown in Fig. 16 differs from the kits of Figs. 14 and 15 in that longitudinal duct 5 encloses a valve element 50 of the type previously described, for example of the type shown in Figs. 8-13B. Endo-urethral prosthesis 1 that can be obtained from such a kit is therefore suitable for treating patients suffering from urinary incontinence, similarly to the endo-urethral prostheses that can be obtained from the kit shown in Fig. 6.

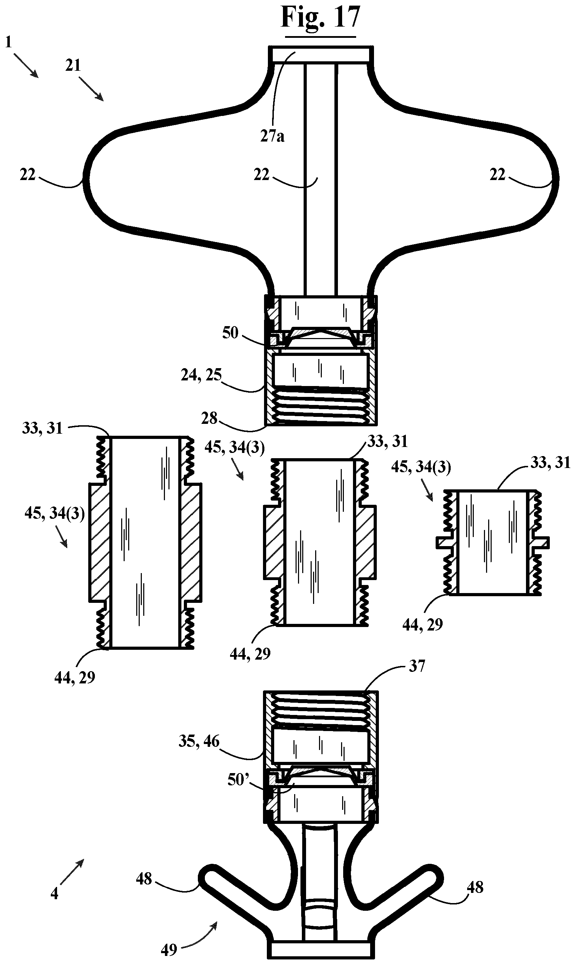

Fig. 17 shows a kit according to another exemplary embodiment, which differs from the kit of Fig. 16 in that it comprises two valve elements 50,50' instead of one. In the embodiment modification shown in the figure, a proximal valve element 50 is arranged at first proximal tubular element 25, thus within proximal part 2, whereas a distal valve element 50' is arranged at distal tubular portion 46, thus within distal part 4. In unshown embodiment modifications of this kit, however, one or both of valve elements 50,50' may be arranged within proximal part 2 or 21-24, or within intermediate part 3, or within distal portion 35-49 or 4 of endo-urethral prosthesis 1.

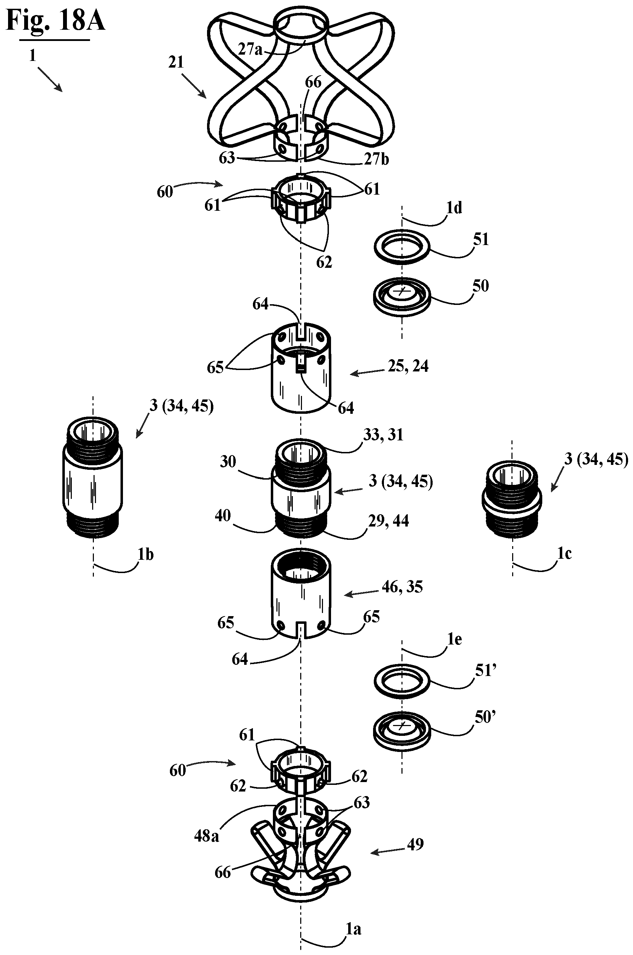

Fig. 18A synoptically shows kits, in an exploded axonometric view, according to modifications of the exemplary embodiments shown in Figs. 14 to 17. In particular, the set of components shown along axes 1a, 1b and 1c constitutes the kit of Figs. 14 and 15. Including also the components shown along axis 1d, the set thus obtained constitutes the kit of Fig. 16, and further including the components shown along axis 1e, the set thus obtained constitutes the kit of Fig. 17.

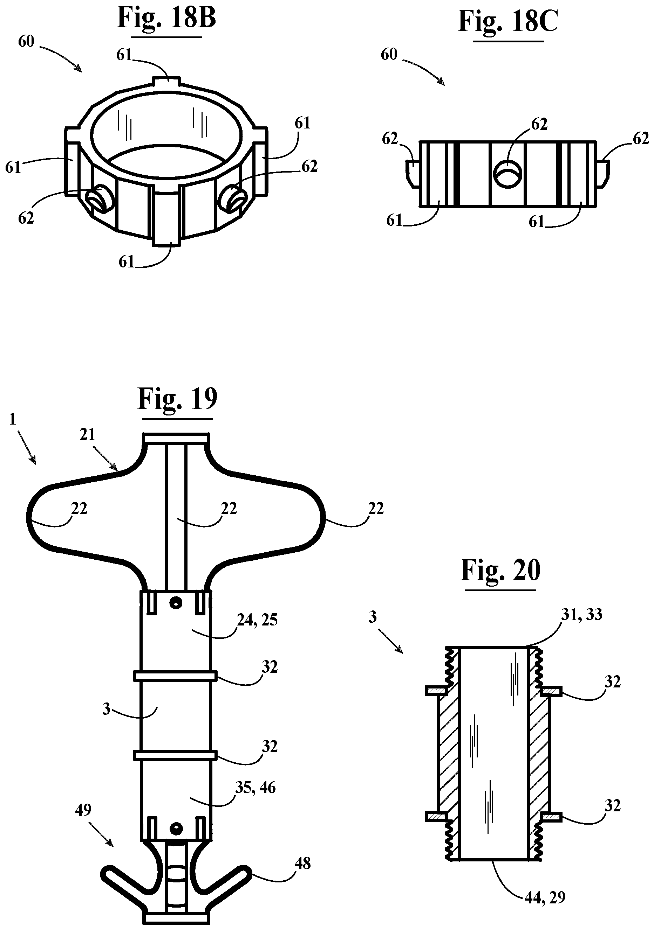

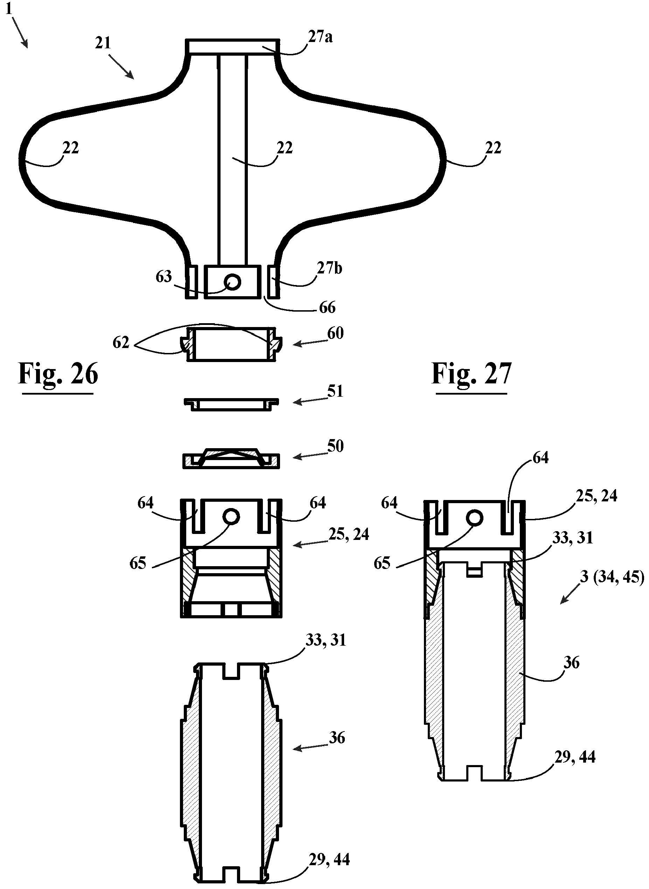

Fig. 18A also shows a method of mounting proximal and distal stents 21 and 49, respectively, to first proximal and distal tubular elements 25,35, as well as a method of mounting valve elements 50,50', when they are present (axes 1d and 1e, respectively). For this purpose, there are provided connecting elements 60, shown in greater detail in Figs. 18B and 18C, comprising a cylinder from the outer surface of which first radial protrusions 61 protrude, preferably uniformly angularly spaced, extending over the entire height of respective connecting element 60, and also second radial protrusions 62 protrude that have a generally circular cross-sectional area and that are arranged circumferentially alternating with first radial protrusions 61, preferably halfway the height of connecting elements 60. First and second radial protrusions 61 and 62 are arranged to engage with through cavities 63, 64 in the same order and between respective flexible ring segments of connecting rings 27b and 48a of proximal and distal stents 21 and 49, and with corresponding through cavities 64,65 made at portions of connection ends of first proximal and distal tubular elements 25,35. In more detail, connecting rings 27b and 48a (see for example Fig. 5) of proximal and distal stents 21 and 49 are radially interposed between the inner surface of the connection end portions of first proximal and distal tubular elements 25,35 and the outer surface of the cylinder of the corresponding connecting element 60. Preferably, as shown in an equivalent embodiment in Fig. 26, second radial protrusions 62 are configured to engage snugly with the through cavities 62 and 65, in particular second radial protrusions 62 are provided with an invitation formed by a chamfer in the direction of insertion of connecting elements 60.

Fig. 19 shows a mounted endo-urethral prosthesis that can be obtained from a kit according to a modification of any of the embodiments of Figs. 14-17, in which intermediate part 3 comprises two deformable radial sealing elements 32, preferably made of a polymeric material, radially protruding at connection ends 31,44 or 33,29. As shown in Fig. 20, radial sealing elements 32 may be flat rings or even, in a modification not shown, simple O-rings.

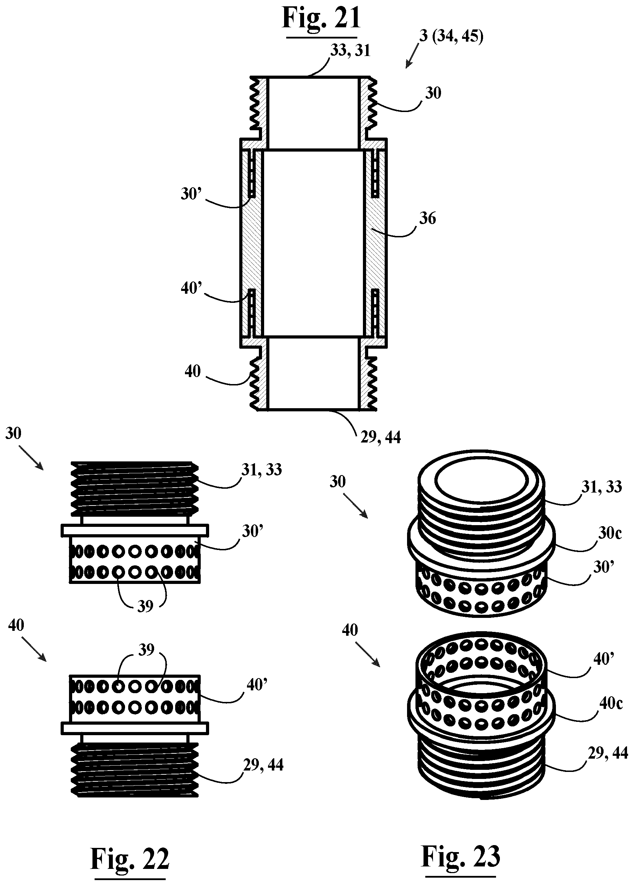

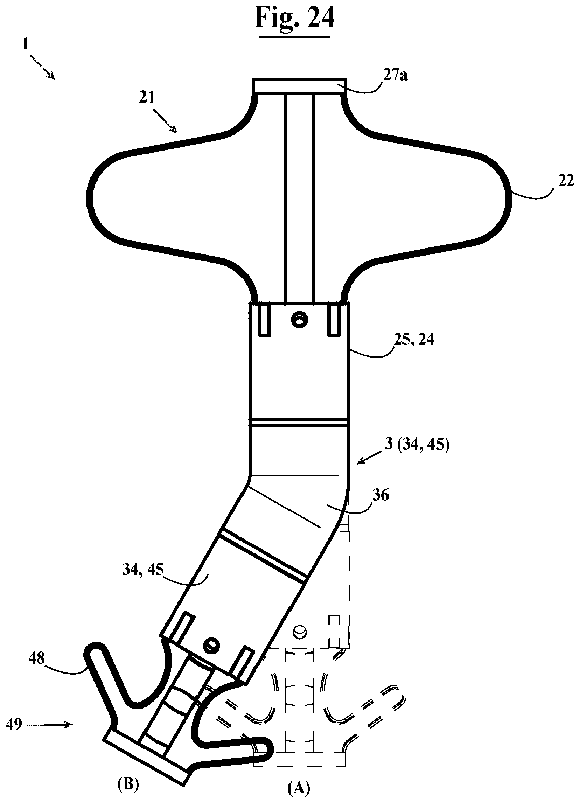

Figs. 21-23 show an exemplary embodiment of intermediate part 3 of any of the kits of Figs. 14-17, wherein intermediate part 3 comprises an axially flexible elongated central body 36, preferably made of a biocompatible polymeric material, for example a medical-grade silicone, and two rigid connection end portions 30,40, preferably made of a metallic material, comprising ends 31 and 44 for connection to proximal part 2 and to first distal tubular element 35 (Fig. 14), or ends 33 and 29 for connection to first proximal tubular element 25 and distal part 4 (Fig. 15). In this way, intermediate part 3 is axially flexible, and therefore also endo-urethral prosthesis 1 is axially flexible. A straight conformation (A) and a bent conformation (B) of such an endo-urethral prosthesis 1 is shown Fig. 24 in dotted line and in solid line, respectively. Connection ends 31,44 or 33,29 are shown, in a non-limiting manner, as threaded ends.

Still with reference to Figs. 21-23, end portions 30,40 comprise cylindrical extensions 30',40' on the side opposite to connection ends 31,44 or 33,29. Cylindrical extensions 30',40' are preferably moulded within polymeric central body 36. Advantageously, cylindrical extensions 30',40' have through grip holes 39 in order to improve the anchoring of end portions 30,40 to polymeric central body 36.

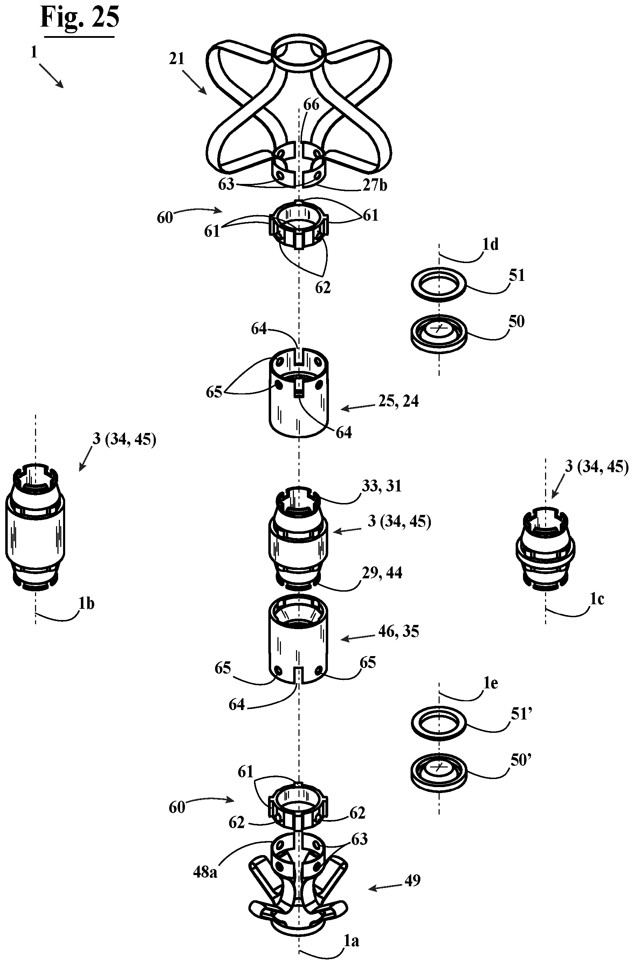

In the previously described figures, connection ends 29,31 of upper proximal part 2 and of lower distal part 4, as well as connection ends 31,44 or 33,29 of intermediate part 3, and corresponding connection ends 28,37 of the parts connected thereto, are represented as threaded connection ends. However, those connection ends may be of different types, in particular they may be snap and interlock connection ends, as shown in Fig. 25, which is a synoptical representation of a plurality of kits equivalent to Fig. 18, and in Fig. 26.

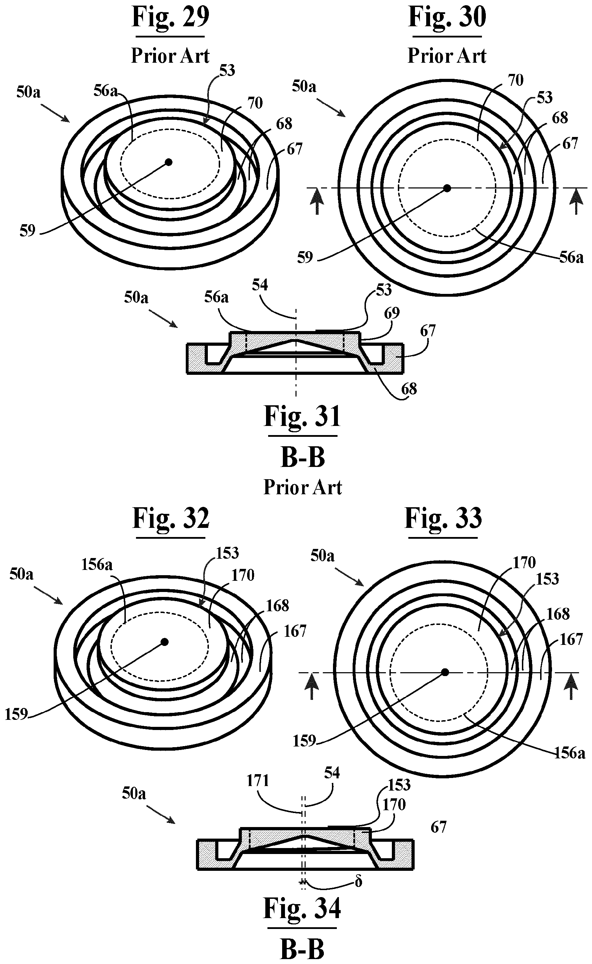

With reference to Figs. 29-47, described hereinafter, an improved valve element 150,250 according to another aspect of the invention. is described. Endo-urethral prosthesis 1, described above, can be equipped of such improved valve element 150,250.

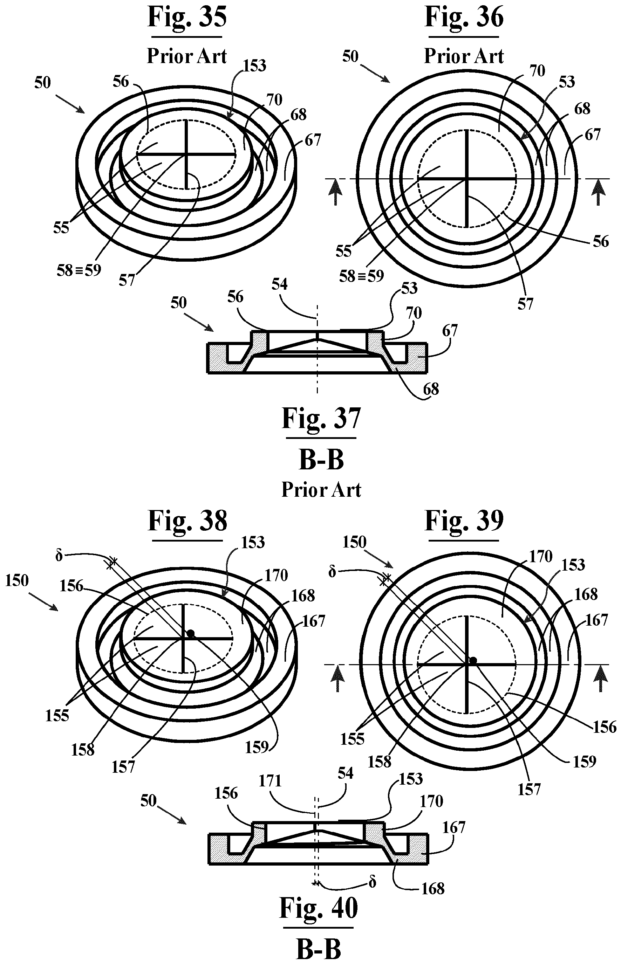

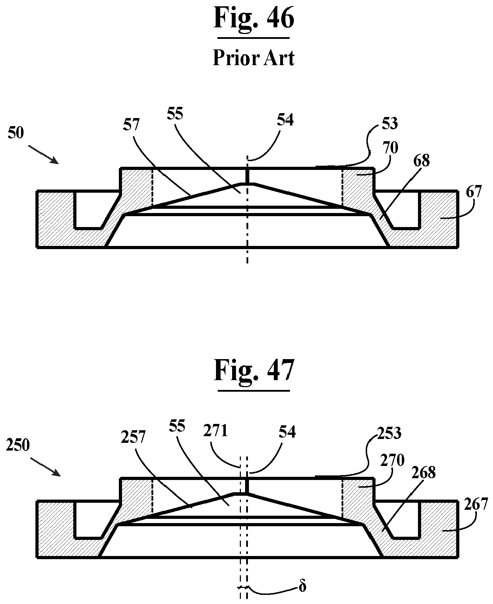

Valve element 150,250 is fluid-tightly arranged within one portion selected between proximal tubular portion 24 and distal tubular portion 46. Similarly to valve elements 50 and 50' described (Figs. 8-13B), valve element 150, shown in Figs. 38-40 and valve element 250, shown in Fig. 47, include a stopper portion 156,256 comprising resilient walls 155,255. Also in this case, when a differential pressure ΔP acting on resilient walls 155,255 exceeds a predetermined opening pressure P* of valve element 150,250 resilient walls 155,255 are configured to be deformed from a rest closed configuration, in which urine 99 cannot flow through longitudinal endo-urethral duct 5, to a forced open configuration of the valve element, in which the urine 99 can flow through longitudinal endo-urethral duct 5. This way, the patient can change the configuration of valve element 150,250 from the closed configuration to the open configuration by causing his/her abdominal pressure to increase above opening pressure P*.

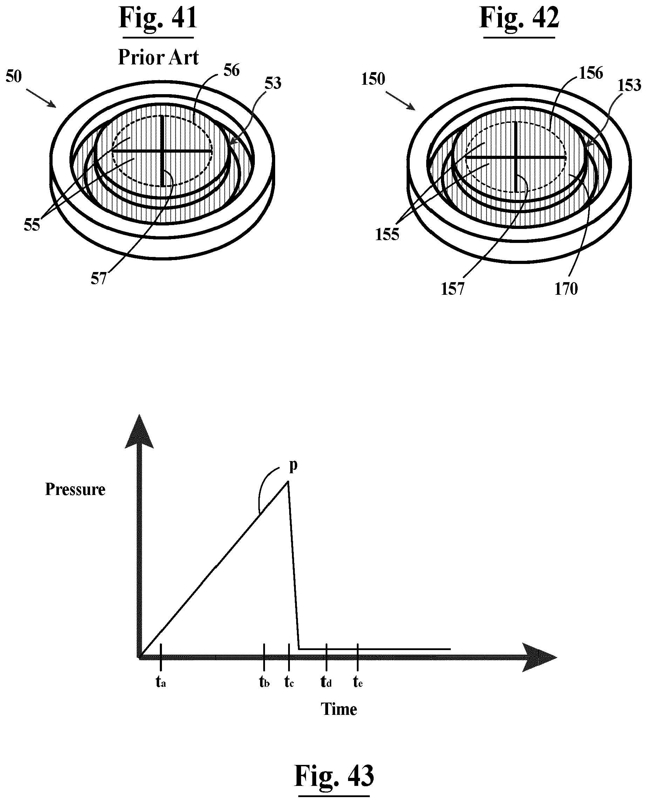

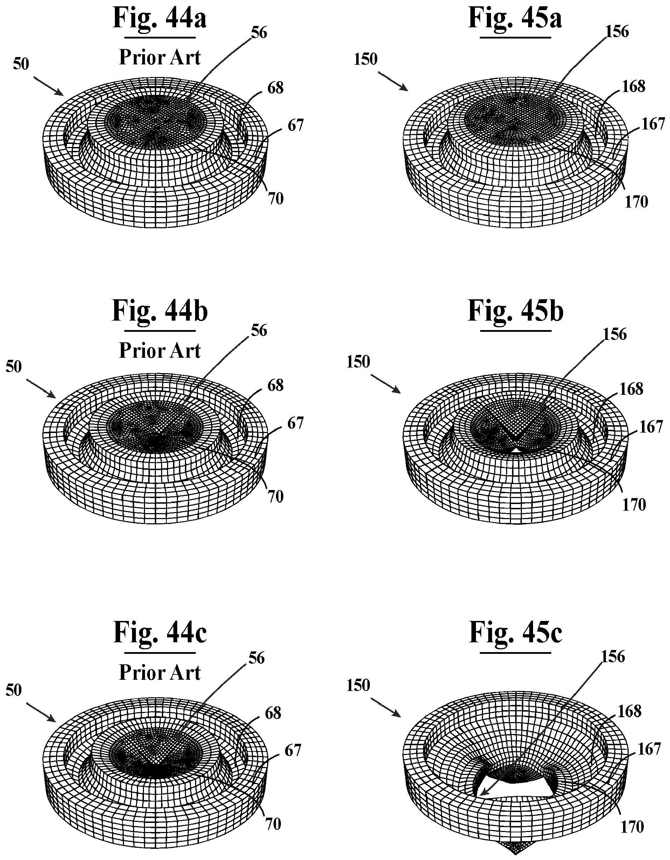

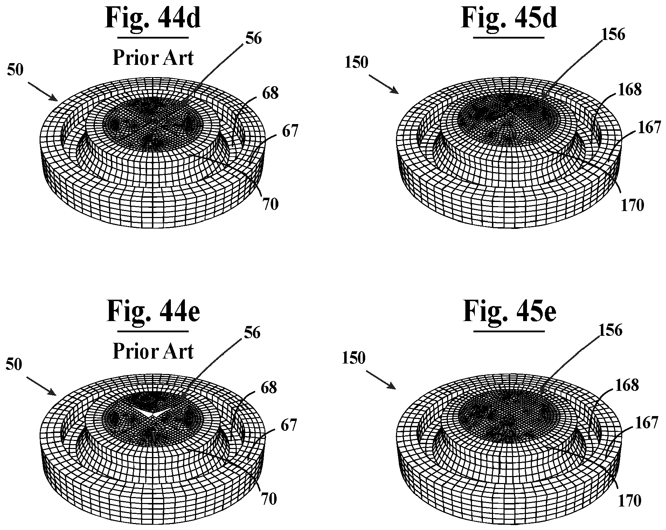

The rest closed configuration and the open forced configuration are shown, for valve element 150, in Figs. 42,45a and in Fig. 45c, respectively.

More in detail, resilient walls 155,255 of stopper portion 156,256, converging to a same convergence point 158,258, are separated from each other by a plurality of through slits 157,257.

In the rest closed configuration (Figs. 42 and 45a), stopper portion 156,256 has a flat shape or a convex shape, for example a dome shape, not shown, with a convexity pointing to the bladder side, i.e. oriented towards proximal stent 21, such that a urine pressure acting on upstream side of stopper portion 156,256 keeps through slits 157,257 closed, provided that the urine pressure does not exceed opening pressure P*. Upon reaching opening pressure P*, stopper portion 156,256 collapses into a concave shape, opposite to the convex shape, in which through slits 157,257 are deformed and stopper portion 156,256 is open as shown in Fig. 45c, so as to allow the flow of urine 99 through longitudinal duct 5 and therefore through urethra 7.

Resilient walls 155,255 are obtained from a flat or convex thin membrane 153,253 by making through slits 157,257 therethrough, preferably, by a punching operation. The same manufacturing procedure can be used for obtaining valve element 50 shown in Figs. 8 to 13B.

Moreover, valve element 150,250 comprises a peripheral cylindric montage portion 167,267 fluid-tightly mounted to an internal wall of at least one tubular portion 24,46, i.e. of longitudinal duct 5. Preferably, peripheral cylindric montage portion 167,267 has circular cross sections. A central point 159,259 of valve element 150,250 is defined with respect to cylindric montage portion 167,267. More precisely, central point 159,259 is the intersection of longitudinal axis 54 of valve element 150,250 (of cylindric montage portion 167,267) with thin membrane 153,253 from which resilient walls 155,255 are obtained by making through slits 157,257, i.e. with a surface - a plan or an axisymmetric shell - to which thin membrane 153,253 can be approximated.

In improved valve elements 150,250, convergence point 158,258, to which through slits 157,257 converge, is shifted from central point 159,259 by a predetermined distance δ. Preferably, distance δ is at least 0,02 mm, in particular, δ is set between 0,02 and 1 mm.

Stopper portion 156,256 preferably comprises a peripheral support disc 170,270 from which resilient walls 155,255 centripetally extend towards central point 159,259. Peripheral support disc 150 corresponds to a peripheral portion of membrane 153,253 that remains unchanged when through slits 157,257 are formed. A connection portion of valve element 150,250 is also provided between peripheral support disc 170,270 and montage portion 167,267 described above.

Figs. 38-40 show a valve element 150 according to an exemplary embodiment, in which peripheral support disc 170 and therefore membrane 153 is concentric to peripheral cylindric montage portion 167, and stopper portion 156 is manufactured eccentrically with respect to membrane 153, therefore the radial width of peripheral support disc 170 changes along the circumference of membrane 153. Figs 32 to 34 show a draft 150' to make valve element 150, i.e. valve element 150 before slits 157 are made to obtain resilient walls 155 from a central portion of membrane 153, in particular, before membrane 153 is punched to make through slits 157. In this case, slits 157 are obtained by positioning a punching device eccentrically with respect to membrane 153. In particular, the cutting edge of a blade of such a punching device, whose length is twice the length of one through slit 157, is positioned eccentrically with respect to membrane 153 and therefore to peripheral cylindric montage portion 167, i.e., with its middle point, not shown, at a distance δ from central point 159 to punch the first two slits as a same linear cut, then is turned by 90° about a rotation axis 171 parallel to longitudinal axis 54 of valve element 150 and passing through the middle point of the punched cut corresponding to first two through slits 157, and after that the last two slits are made. Convergence point 158 coincides with the intersection of rotation axis 171 with membrane 153, and is at a distance δ from central point 159.

In valve element 150, the eccentricity of slits 157 and membrane 153, or peripheral cylindric montage portion 167, or valve element 150 itself, is obtained by eccentrically working -punching- a same symmetrical draft 50a that would have been used to make symmetrical valve element 50. For comparison, see figs. 29-31 and 35-37 in the light of the above description, in which rotation axis 71 of the punching device and converging point 58 of through slits 155 coincide with longitudinal axis 54 of draft 5' and, finally, of symmetrical valve element 50.

Figs. 41 and 42 show valve elements 50 and 150, according to the prior art and to the above embodiment of the invention, respectively, and are the same pictures as Figs. 35 and 38, apart from the hatched areas, corresponding to the surfaces on which urine pressure P acts. A typical trend of urine pressure in a cycle involving the accumulation of urine 99 within bladder 6 and the subsequent release of the urine, due to the opening of the valve element, is shown in Fig. 43, in which specific instants tₐ-tₑ of the cycle are indicated.

Figs 44a-e and 45a-e show how typically resilient walls 55,155 of valve elements 50 and 150, according to the prior art and to the invention change their position as urine pressure P changes as indicated in Fig. 43, respectively. Figs 44a-e and 45a-e correspond to instants tₐ-tₑ, in the same order. At time tₐ, when bladder 6 is empty and urine pressure P is 0 of far lower than opening pressure P*, both valve element 50 and 150 are in the closed configuration, i.e. resilient walls 55,155 are fully in contact with one another by longitudinal slits 57,157 (Figs. 44a,45a). At time t_{b}, when urine pressure has nearly attained opening pressure P* (corresponding to the maximum opening of the valve), prior art valve element 44 is still substantially closed, while valve element 45 already shows a small, partial opening of valve element 150, which bear witness to a quicker response capability of valve element 150 with respect to prior art valve element 50. At time tc, when urine pressure has attained opening pressure P*, valve element 150 is fully open, while prior art valve element 50 can show a reduced passageway with respect to valve element 50, and urine pressure P fall down to zero value, time t_{d} and tₑ, and resilient walls 55,155 are elastically recalled to the respective rest closed configurations. However, as shown in Fig. 44e, resilient walls 55 of prior art valve 50 can get stuck together at the end of the cycle, and prior art valve 50 can show a residual passageway even when no urine is present above to stopper elements 56, which can cause a urine leakage, while this is never the case with valve element 150, which is completely closed, see Fig. 45e.

Fig. 47 shows a valve element 250 according to another exemplary embodiment, in which peripheral support disc 270 and therefore membrane 253 is eccentric with respect to peripheral cylindric montage portion 267, and stopper portion 256 is manufactured concentrically to membrane 253, therefore the radial width of peripheral support disc 270 is unchanged along the circumference of membrane 253. In this case, slits 257 can be obtained by positioning a punching device concentrically with respect to membrane 253. In particular, the cutting edge of a blade of the punching device, is positioned concentrically with respect to membrane 253 and therefore eccentrically to peripheral cylindric montage portion 267, i.e., with its middle point, not shown, at a distance δ from central point 259 to punch the first two slits as a same linear cut, then is turned by 90° about a rotation axis 271 coincident to longitudinal axis 54 of valve element 250 and passing through the middle point of the punched cut corresponding to first two through slits 257, and after that the last two slits are made. Also in this case, convergence point 258 coincides with the intersection of rotation axis 272 with membrane 253, and is at a distance δ from central point 259.

Concerning the displacement of resilient walls 255 and the opening and reclosure of valve element 250 due to the increase and subsequent decrease of urine pressure acting on valve element 250, shown in Fig. 43, a behaviour similar to the behaviour of valve element 150 has been observed, as depicted in Figs. 45a to 45e.

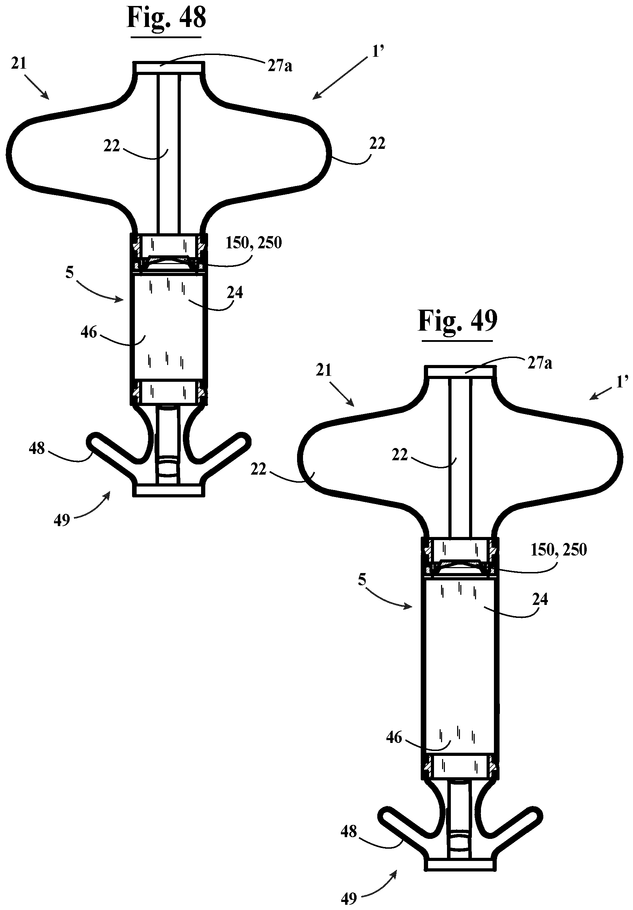

According to a further aspect of the invention, there is provided a one-piece endo-urethral prosthesis 1', shown in Figs. 48 and 49. including improved valve element 150,250 described above.

Endo-urethral prosthesis 1' comprises a proximal part 2 and a distal part 4 including proximal and distal tubular portions 24,46, integral to each other and having a predetermined radial stiffness. Proximal and distal tubular portions 24,46 are configured to be arranged within a patient's urethra 7, and proximal and distal stents 21,49, configured to be reversibly fixed to an inner wall of a urethra-vesical lumen 6-7 and of urethra 7, respectively, of a female or male patient suffering from urinary incontinence (see Figs. 1 and 2, respectively). Proximal and distal tubular portions 24,46 form together a longitudinal endo-urethral duct 5 that is rigid enough to maintain its shape under the implantation conditions.

Advantageously, the surfaces of endo-urethral prosthesis 1 or 1' that will come into contact with the patient's tissues have a coating of a biocompatible material, preferably selected among hydrophilic medical-grade silicones and/or hydrogel with anti-fouling and anti-bacterial properties, in particular selected from the group consisting of a zwitterion polymer, polyethylene glycol, a combination thereof. In particular, the hydrogel can be combined with polydopamine to improve long-term adhesion on coated surfaces.

The above description of exemplary embodiments and modifications thereof of the invention is capable of showing the invention from a conceptual point of view in such a way that others, using the known technique, will be able to modify and/or adapt in various applications those specific embodiments and modifications thereof without further research and without departing from the inventive concept, and, therefore, it is understood that such adaptations and modifications will be considered as equivalents of the embodiments and modifications thereof. The means and materials for putting into practice the functions described herein may be of various kinds without departing from the scope of the invention. It is understood that the expressions or terminology used are purely descriptive and, therefore, not limitative.

## Claims

1. A kit for an endo-urethral prosthesis (1), comprising a proximal part (2) and a distal part (4) for said endo-urethral prosthesis (1),
wherein said proximal part (2) comprises:
- a proximal tubular portion (24) configured to be arranged within a patient's urethra (7), and
- a proximal stent (21) configured to be reversibly fixed to an inner wall (8) of a urethra-vesical lumen (6-7);
wherein said distal part (4) comprises:
- a distal tubular portion (46) configured to be arranged within said urethra (7), and
- a distal stent (49) configured to be reversibly fixed to said inner wall (8) of said urethra (7),
said proximal tubular portion (24) and said distal tubular portion (46) having a predetermined radial stiffness,
wherein said proximal tubular portion (24) and said distal tubular portion (46) have, on sides opposite to said proximal and distal stents (21,49), respectively, a proximal connection end (29) and a distal connection end (31) configured to be fluid-tightly connected to each other and so to connect said proximal part (2) and said distal part (4),
so as to define a single longitudinal duct (5) arranged to convey urine (99), said longitudinal duct consisting of said proximal tubular portion (24) and of said distal tubular portion (46),
and wherein either said proximal part (2) or said distal part (4) is a single part (2, 4) of an assigned length (L₂, L₄), and the other part (4,2), either distal or proximal, is provided as a range of two or more parts of different lengths (L₄, L₂), configured to be assembled selectively with said proximal or distal part (2,4) provided as a single specimen of an assigned length (L₂, L₄), respectively, so as to make said endo-urethral prosthesis (1) of two or more overall lengths (L₁), in order to select one therefrom most appropriate to a patient's urethra.

2. The kit according to claim 1, wherein said distal part (4) is provided as a range of two or more parts of different lengths (L₄) and said distal tubular portion (46) is split into a first distal tubular element (35) of an assigned length (L₃₅), and a second distal tubular element (45) provided as a range of two or more elements of different lengths (L₄₅), said second distal tubular element (45) comprising said distal connection end (31), wherein said first distal tubular element (35) and said second distal tubular element (45) have respective mutual fluid-tight connection ends (37,44), said second distal tubular element (45) forming an intermediate part (3) of said endo-urethral prosthesis (1).

3. The kit according to claim 1, wherein said proximal part (2), is provided as a range of two or more parts of different lengths (L₂) and said proximal tubular portion (24) is split into a first proximal tubular element (25) of an assigned length (L₂₅) and a second proximal tubular element (34) provided as a range of two or more elements of different lengths (L₃₄), said second proximal tubular element (34) comprising said proximal connection end (29), wherein said first proximal tubular element (25) and said second proximal tubular element (34) have respective mutual fluid-tight connection ends (28,33), said second proximal tubular element (34) forming an intermediate part (3) of said endo-urethral prosthesis (1).

4. The kit according to claim 1, wherein a valve element (50,150,250) is fluid-tightly arranged within at least one portion selected between said proximal tubular portion (24) and said distal tubular portion (46), said valve element (50,150,250) including a stopper portion (56,156,256), wherein said stopper portion (56,156,256) comprises resilient walls (55,155,255) configured to be deformed:
- from a rest closed configuration of said valve element (50,150,250), in which said urine (99) cannot flow through said longitudinal duct (5),
- to a forced open configuration of said valve element (50,150,250), in which said urine (99) can flow through said longitudinal duct (5),
when a differential pressure (ΔP) acting on said resilient walls (55,155,255) exceeds a predetermined opening pressure (P*) of said valve element (50,150,250),
so that said patient can change said configuration of said valve element (50,150,250) from said closed configuration to said open configuration by causing his/her abdominal pressure to increase.

5. The kit according to claim 4, wherein said resilient walls (55,155,255) of said stopper portion (56,156,256) are separated from each other by a plurality of through slits (57), wherein
- in said closed configuration, said stopper portion (56,156,256) has a shape selected between a flat shape and a convex shape with a convexity oriented towards said proximal stent (21), in such a way that a urine pressure acting on said stopper portion (56,156,256) keeps said through slits (57,157,257) closed;
- in said open configuration, said stopper portion (56,156,256) has a concave shape, in particular opposite to said convex shape, wherein said through slits (57,157,257) are deformed and open, and is configured to allow a flow of said urine (99),
wherein, in said closed configuration, said stopper portion (56,156,256) is configured to withstand said urine pressure until said urine pressure does not exceed said opening pressure (P*), so that, upon exceeding said opening pressure (P*), said stopper portion (56,156,256) collapses into said concave shape,
in particular, said stopper portion (56,156,256) has a dome shape with said through slits (57,157,257) converging to a central region (58,158,258) of said stopper portion (56,156,256).

6. The kit according to claim 5,
wherein said valve element (150,250) comprises a peripheral cylindric montage portion (167,267) fluid-tightly mounted to an internal wall of said at least one tubular portion (24,46),
wherein said stopper portion (156,256) of said valve element (150,250) has a central point (159,259) which is the centre with respect to the cylindric montage portion (167,267),
and wherein said through slits (157,257) converge to a same convergence point (158,258) that is shifted from said central point (159,259) by a predetermined distance (δ).

7. The kit according to claim 6,
wherein said stopper portion (156,256) comprises a peripheral support disc (170,270) from which said resilient walls (155,255) centripetally extend towards said central point (159,259),
wherein said valve element (150,250) comprises a connection portion (168,268) between said peripheral support disc (170,270) and said montage portion (167,267),
wherein said peripheral support disc (170,270) is selected between:
- a peripheral support disc (170,270) concentric to said peripheral cylindric montage portion (167,267), and said stopper portion is manufactured eccentrically to said peripheral support disc (170,270);
- a peripheral support disc (170,270) eccentric to said peripheral cylindric montage portion (167,267), and said stopper portion is manufactured concentrically to said peripheral support disc (170,270).

8. The kit according to claim 2 or 3, wherein two annular sealing elements (32) protrude from an outer surface of said intermediate part (3),
in particular said annular sealing elements (32) are arranged at said connection ends (31,44;33,29) of said intermediate part (3).

9. The kit according to claim 2 or 3, wherein said intermediate part (3) of said endo-urethral prosthesis (1) is axially flexible, i.e., it is configured to move:
- from a straight conformation, in which said distal part (4) is aligned with said proximal part (2)
- to a bent conformation, in which said proximal portion (2) and said distal portion (4) are connected by an elbow portion (3),
in particular, said intermediate part (3) is at least in part made of a biocompatible flexible polymeric material (36), more in particular, of a medical-grade silicone.

10. The kit according to claim 2 or 3, wherein said intermediate part (3) of said endo-urethral prosthesis (1) comprises:
- two rigid connection end portions (30,40) for connection to:
- said proximal part (2) and said first distal tubular element (35), or
- said first proximal tubular element (25) and said distal part (4);
- a central axially flexible tubular portion (36), made of a polymeric material having a predetermined elasticity modulus,
in particular, said two rigid connection end portions (30,40) comprise respective cylindrical extensions (30',40') moulded within a thickness of said central axially flexible tubular portion (36),
in particular, said cylindrical extensions (30',40') have grip-enhancing through-holes (39) for a material of said central axially flexible tubular portion (36).

11. The kit according to claim 4, wherein said valve element is a proximal valve element (50,150,250) and a distal valve element (50') is fluid-tightly arranged within said longitudinal duct (5), distally with respect to said proximal valve element (50,150,250),
in particular said proximal valve element (50,150,250) has a reclosing pressure higher than the opening pressure of said distal valve element (50'), so as to clear a portion (41) of said longitudinal canal (5) between said proximal and distal valve elements (50,150,250,50') from said urine (99), after evacuating said urine (99).

12. The kit according to claim 1, further comprising a coating of a biocompatible material on the surfaces of said endo-urethral prosthesis (1) that, in use, are in contact with said patient's tissues,
wherein said biocompatible coating material is selected from the group consisting of:
- a hydrophilic medical-grade silicone;
- a hydrogel with anti-fouling and anti-bacterial properties, in particular selected from the group consisting of a zwitterion polymer, polyethylene glycol, a combination thereof
in particular, said hydrogel is combined with polydopamine so as to improve long-time adhesion of said hydrogel to said surfaces.

13. An endo-urethral prosthesis (1') comprising a proximal part (2) and a distal part (4),
wherein said proximal part (2) comprises:
- a proximal tubular portion (24) configured to be arranged within a patient's urethra (7), and
- a proximal stent (21) configured to be reversibly fixed to an inner wall (8) of a urethra-vesical lumen (6-7);
wherein said distal part (4) comprises:
- a distal tubular portion (46) configured to be arranged within said urethra (7), and
- a distal stent (49) configured to be reversibly fixed to said inner wall (8) of said urethra (7),
said proximal tubular portion (24) and SAID distal tubular portion (46) having a predetermined radial stiffness and defining a longitudinal duct (5) arranged to convey urine (99),
wherein a valve element (150,250) is fluid-tightly arranged within at least one portion between said proximal tubular portion (24) and said distal tubular portion (46), said valve element (150,250) including a stopper portion (156,256), wherein said stopper portion (156,256) comprises resilient walls (155,255) that are configured to be deformed:
- from a rest closed configuration of said valve element (150,250), in which said urine (99) cannot flow through said longitudinal duct (5),
- to a forced open configuration of said valve element (150,250), in which said urine (99) can flow through said longitudinal duct (5),
when a differential pressure (ΔP) acting on said resilient walls (155,255) exceeds a predetermined opening pressure (P*) of said valve element (150,250),
so that said patient can change said configuration of said valve element (150,250) from said closed configuration to said open configuration by causing his/her own abdominal pressure to increase;
wherein said resilient walls (155,255) of said stopper portion (156,256) are separated from each other by a plurality of through slits (157,257), wherein:
- in said closed configuration, said stopper portion (156,256) has a shape selected between a flat shape and a convex shape with a convexity oriented towards said proximal stent (21), in such a way that a urine pressure acting on said stopper portion (156,256) keeps said through slits (157,257) closed;
- in said open configuration, said stopper portion (156,256) has a concave shape, in particular opposite to said convex shape, wherein said through slits (157,257) are deformed and open, and is configured to allow a flow of said urine (99),
wherein, in said closed configuration, said stopper portion (156,256) is configured to withstand said urine pressure until said urine pressure does not exceed said opening pressure (P*), so that, upon exceeding said opening pressure (P*), said stopper portion (156,256) collapses into said concave shape,
wherein said valve element (150,250) comprises a peripheral cylindric montage portion (167,267) fluid-tightly mounted to an internal wall of said at least one tubular portion (24,46):
wherein said stopper portion (156,256) of said valve element (150,250) has a central point (159,259) which is the centre with respect to the cylindric montage portion (167,267),
**characterized in that** said through slits (157,257) converge to a same convergence point (158,258) that is shifted from said central point (159,259) by a predetermined distance (δ).

14. The endo-urethral prosthesis (1') according to claim 12,
wherein said stopper portion (156,256) comprises a peripheral support disc (170,270) from which said resilient walls (155,255) centripetally extend towards said central point (159,259),
wherein said valve element (150,250) comprises a connection portion (168,268) between said peripheral support disc (170,270) and said montage portion (167,267),
wherein said peripheral support disc (170,270) is selected between:
- a peripheral support disc (170,270) concentric to said peripheral cylindric montage portion (167,267), and said stopper portion is manufactured eccentrically to said peripheral support disc (170,270);
- a peripheral support disc (170,270) eccentric to said peripheral cylindric montage portion (167,267), and said stopper portion is manufactured concentrically to said peripheral support disc (170,270).

15. The endo-urethral prosthesis (1') according to claim 12, wherein said convergence point (158,258) and said central point (159,259) are at a distance (δ) of at least 0,02 mm.
